# EUROPÄISCHE PATENTANMELDUNG

(11) **EP 2 022 791 A1**
(43) Veröffentlichungstag der Anmeldung: **11.02.2009**
(21) Anmeldenummer: 07015301.0
(22) Anmeldetag: 03.08.2007
(51) Int. Cl.: C07D 413/04, C07D 413/14, A01N 47/36, A01N 47/38

(54) **Herbizide dioxazinyl-substituierte Furylsulfonylaminocarbonylverbindungen**

(71) Anmelder: Bayer CropScience AG, 40789 Monheim (DE)
(72) Erfinder: Gesing, Ernst R.F., 40699 Erkrath-Hochdahl (DE); Müller, Klaus-Helmut, 40593 Düsseldorf (DE); Waldraff, Christian, 61118 Bad Vilbel (DE); Kehne, Heinz, 65719 Hofheim im Taunus (DE); Feucht, Dieter, 65760 Eschborn (DE); Hills, Martin, 65510 Idstein (DE); Rosinger, Chris, 65719 Hofheim im Taunus (DE); Dittgen, Jan, 60316 Frankfurt (DE)

(57) **Zusammenfassung**

Die Erfindung betrifft neue substituierte Furylsulfonylaminocarbonylverbindungen der allgemeinen Formel (I), 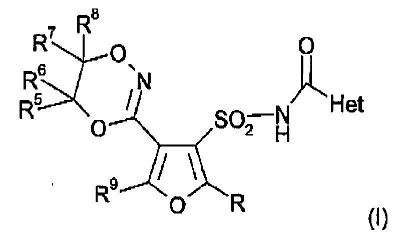 in welcher R, R⁹, Het und R⁵ bis R⁸ die in der Beschreibung angegebene Bedeutung haben,

Verfahren und Zwischenprodukte zu ihrer Herstellung und ihre Verwendung als Herbizide.

## Beschreibung

### Dioxazinyl-substituierte Furylsulfonylaminocarbonylverbindungen

Die Erfindung betrifft neue substituierte Furylsulfonylaminocarbonylverbindungen, Verfahren und Zwischenprodukte zu ihrer Herstellung und ihre Verwendung als Herbizide.

Es ist bereits bekannt, dass bestimmte Dioxazinyl-substituierte Thienylsulfonylaminocarbonylverbindungen herbizide Eigenschaften aufweisen (vgl. US 5,476,936, WO 2006012982). Die herbizide Wirksamkeit dieser bekannten Verbindungen ist jedoch nicht in allen Belangen zufriedenstellend.

Es wurden nun die neuen substituierten Furylsulfonylaminocarbonylverbindungen der allgemeinen Formel (I) 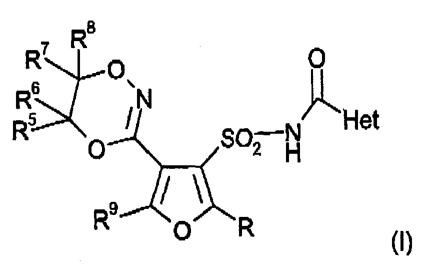 in welcher
- Het: für 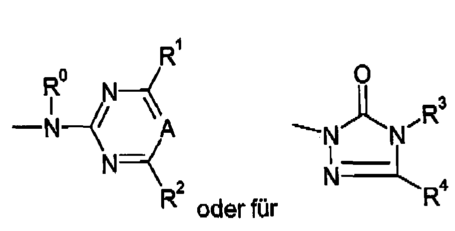
steht,
- A: für Stickstoff oder eine CH-Gruppierung steht,
- R: für Wasserstoff, Cyano, Nitro, Halogen, für jeweils gegebenenfalls durch Cyano, Halogen oder (C₁-C₄)-Alkoxy substituiertes Alkyl, Alkoxy, Alkoxy-carbonyl, Alkylthio, Alkylsutfinyl oder Alkylsulfonyl mit jeweils 1 bis 6 Kohlenstoffatomen in der Alkylgruppe, oder für jeweils gegebenenfalls durch Cyano oder Halogen substituiertes Alkenyl, Alkinyl, Alkenyloxy oder Alkinyloxy mit jeweils 2 bis 6 Kohlenstoffatomen in der Alkenyl- oder Alkinylgruppe steht,
- R⁰: für Wasserstoff, oder für (C₁-C₄)-Alkyl steht,
- R¹: für Wasserstoff, für Halogen, für jeweils gegebenenfalls durch Cyano, Halogen oder (C₁-C₄)-Alkoxy substituiertes Alkyl, Alkoxy, Alkylthio, Alkylamino oder Dialkylamino mit jeweils 1 bis 4 Kohlenstoffatomen in den Alkylgruppen, oder für jeweils gegebenenfalls durch Cyano, Halogen, (C₁-C₄)-Alkyl oder (C₁-C₄)-Alkoxy substituiertes Phenoxy, Oxetanyloxy, Furyloxy oder Tetrahydrofuryloxy steht,
- R²: für Wasserstoff, für Halogen, für jeweils gegebenenfalls durch Cyano, Halogen oder (C₁-C₄)-Alkoxy substituiertes Alkyl, Alkoxy, Alkylthio, Alkylamino oder Dialkylamino mit jeweils 1 bis 4 Kohlenstoffatomen in den Alkylgruppen, oder für jeweils gegebenenfalls durch Cyano, Halogen, (C₁-C₄)-Alkyl oder (C₁-C₄)-Alkoxy substituiertes Phenoxy, Oxetanyloxy, Furyloxy oder Tetrahydrofuryloxy steht,
- R³: für Wasserstoff, Hydroxy, Amino, Cyano, für (C₂-C₁₀)-Alkylidenamino, für gegebenenfalls durch Fluor, Chlor, Brom, Cyano, (C₁-C₄)-Alkoxy, (C₁-C₄)-Alkyl-carbonyl oder (C₁-C₄)-Alkoxy-carbonyl substituiertes Alkyl mit 1 bis 6 Kohlen-stoffatomen, für jeweils gegebenenfalls durch Fluor, Chlor und/oder Brom substituiertes Alkenyl oder Alkinyl mit jeweils 2 bis 6 Kohlenstoffatomen, für jeweils gegebenenfalls durch Fluor, Chlor, Brom, Cyano, (C₁-C₄)-Alkoxy oder (C₁-C₄)-Alkoxy-carbonyl substituiertes Alkoxy, Alkylamino oder Alkyl-carbonyl-amino mit jeweils 1 bis 6 Kohlenstoffatomen in der Alkylgruppe, für Alkenyloxy mit 3 bis 6 Kohlenstoffatomen, für Dialkylamino mit jeweils 1 bis 4 Kohlenstoffatomen in den Alkylgruppen, für jeweils gegebenenfalls durch Fluor, Chlor, Brom, Cyano und/oder (C₁-C₄)-Alkyl substituiertes Cycloalkyl, Cyclo-alkylamino oder Cycloalkylalkyl mit jeweils 3 bis 6 Kohlenstoffatomen in der Alkylgruppe und gegebenenfalls 1 bis 4 Kohlenstoffatomen im Alkylteil, oder für jeweils gegebenenfalls durch Fluor, Chlor, Brom, Cyano, Nitro, (C₁-C₄)-Alkyl, Trifluormethyl und/oder (C₁-C₄)-Alkoxy substituiertes Aryl oder Arylalkyl mit jeweils 6 oder 10 Kohlenstoffatomen in der Arylgruppe und gegebenenfalls 1 bis 4 Kohlenstoffatomen im Alkylteil steht,
- R⁴: für Wasserstoff, Hydroxy, Mercapto, Amino, Cyano, Fluor, Chlor, Brom, lod, für gegebenenfalls durch Fluor, Chlor, Brom, Cyano, (C₁-C₄)-Alkoxy, (C₁-C₄)-Alkyl-carbonyl oder (C₁-C₄)-Alkoxy-carbonyl substituiertes Alkyl mit 1 bis 6 Kohlenstoffatomen, für jeweils gegebenenfalls durch Fluor, Chlor und/oder Brom substituiertes Alkenyl oder Alkinyl mit jeweils 2 bis 6 Kohlenstoffatomen, für jeweils gegebenenfalls durch Fluor, Chlor, Cyano, (C₁-C₄)-Alkoxy oder (C₁-C₄)-Alkoxy-carbonyl substituiertes Alkoxy, Alkylthio, Alkylamino oder Alkyl-carbonylamino mit jeweils 1 bis 6 Kohlenstoffatomen in der Alkylgruppe, für Alkenyloxy, Alkinyloxy, Alkenylthio, Alkinylthio, Alkenylamino oder Alkinyl-amino mit jeweils 3 bis 6 Kohlenstoffatomen in der Alkenyl- oder Alkinyl-gruppe, für Dialkylamino mit jeweils 1 bis 4 Kohlenstoffatomen in den Alkyl-gruppen, für jeweils gegebenenfalls durch Fluor, Chlor, Brom, Cyano und/oder (C₁-C₄)-Alkyl substituiertes Cycloalkyl, Cycloalkenyl, Cycloalkyloxy, Cycloalkylthio, Cycloalkylamino, Cycloalkylalkyl, Cycloalkylalkoxy, Cycloalkylalkylthio oder Cycloalkylalkylamino mit jeweils 3 bis 6 Kohlenstoffatomen in der Cycloalkyl- bzw. Cycloalkenylgruppe und gegebenenfalls 1 bis 4 Kohlenstoffatomen im Alkylteil, oder für jeweils gegebenenfalls durch Fluor, Chlor, Brom, Cyano, Nitro, (C₁-C₄)-Alkyl, Trifluormethyl, (C₁-C₄)-Alkoxy und/oder (C₁-C₄)-Alkoxy-carbonyl substituiertes Aryl, Arylalkyl, Aryloxy, Arylalkoxy, Arylthio, Arylalkylthio, Arylamino oder Arylalkylamino mit jeweils 6 oder 10 Kohlenstoffatomen in der Arylgruppe und gegebenenfalls 1 bis 4 Kohlenstoffatomen im Alkylteil steht, und
- R⁵, R⁶, R⁷ und R⁸: unabhängig voneinander für Wasserstoff, Halogen, Cyano oder Thiocyanato oder für gegebenenfalls durch Halogen substituiertes Alkyl, Alkoxy, Alkylthio, Alkylsulfinyl, Alkylsulfonyl Alkylamino, Alkylcarbonyl, Alkoxycarbonyl oder Alkylaminocarbonyl mit jeweils 1 bis 3 Kohlenstoffatomen im Alkylteil stehen,
- R⁹: für Wasserstoff, Cyano, Nitro, Halogen, für jeweils gegebenenfalls durch Cyano, Halogen oder C₁-C₄-Alkoxy substituiertes Alkyl, Alkoxy, Alkoxy-carbonyl, Alkylthio, Alkylsulfinyl oder Alkylsulfonyl mit jeweils 1 bis 6 Kohlenstoffatomen in der Alkylgruppe, oder für jeweils gegebenenfalls durch Cyano oder Halogen substituiertes Alkenyl, Alkinyl, Alkenyloxy oder Alkinyloxy mit jeweils 2 bis 6 Kohlenstoffatomen in der Alkenyl- oder Alkinylgruppe steht,
sowie Salze von Verbindungen der Formel (I) gefunden.

Gesättigte oder ungesättigte Kohlenwasserstoffgruppierungen, wie Alkyl, Alkenyl oder Alkinyl, sind - auch in Verknüpfungen mit Heteroatomen, wie in Alkoxy - soweit möglich jeweils geradkettig oder verzweigt.

Gegebenenfalls substituierte Reste können einfach oder mehrfach substituiert sein,
wobei bei Mehrfachsubstitution die Substituenten gleich oder verschieden sein können.
- R: steht bevorzugt für Wasserstoff, Cyano, Fluor, Chlor, Brom, für jeweils gegebenenfalls durch Cyano, Fluor, Chlor, Methoxy oder Ethoxy substituiertes Methyl, Ethyl, n- oder i-Propyl, n-, i-, s- oder t-Butyl, Methoxy, Ethoxy, n- oder i-Propoxy, Methoxycarbonyl, Ethoxycarbonyl, n- oder i-Propoxycarbonyl, Methylthio, Ethylthio, n- oder 1-Propylthio, Methylsulfinyl, Ethylsulfinyl, Methyl-sulfonyl oder Ethylsulfonyl, oder für jeweils gegebenenfalls durch Cyano, Fluor oder Chlor substituiertes Propenyl, Butenyl, Propinyl, Butinyl, Propenyloxy, Butenyloxy, Propinyloxy oder Butinyloxy.
- R: steht besonders bevorzugt für Fluor, Chlor, Brom, für jeweils gegebenenfalls durch Cyano, Fluor, Chlor, Methoxy oder Ethoxy substituiertes Methyl, Ethyl, n- oder i-Propyl, n-, i-, s- oder t-Butyl, Methoxy, Ethoxy, n- oder i-Propoxy.
- R: steht am meisten bevorzugt für Methyl, Ethyl, n- oder i-Propyl.
- R⁰: steht bevorzugt für Wasserstoff.
- R¹: steht bevorzugt für Wasserstoff, Fluor, Chlor, Brom, lod, oder für jeweils gegebenenfalls durch Cyano, Fluor, Chlor, Methoxy oder Ethoxy substituiertes Methyl, Ethyl, n- oder i-Propyl, Methoxy, Ethoxy, n- oder i-Propoxy, Methylthio, Ethylthio, n- oder 1-Propylthlo, Methylamino, Ethylamino, n- oder i-Propyl-amino, Dimethylamino oder Diethylamino.
- R¹: steht besonders bevorzugt für Fluor, Chlor, Brom, oder lod, oder für gegebenenfalls durch Fluor oder Chlor substituiertes Methyl, Ethyl, n- oder i-Propyl, Methoxy, Ethoxy, n- oder i-Propoxy oder für Dimethylamino.
- R²: steht bevorzugt für Fluor, Chlor, Brom, lod oder für jeweils gegebenenfalls durch Cyano, Fluor, Chlor, Methoxy oder Ethoxy substituiertes Methyl, Ethyl, n- oder i-Propyl, Methoxy, Ethoxy, n- oder i-Propoxy, Methylthio, Ethylthio, n- oder i-Propylthio, Methylamino, Ethylamino, n- oder i-Propylamino, Dimethyl-amino oder Diethylamino.
- R²: steht besonders bevorzugt für Fluor, Chlor, Brom, oder lod, oder für gegebenenfalls durch Fluor oder Chlor substituiertes Methyl, Ethyl, n- oder i-Propyl, Methoxy, Ethoxy, n- oder i-Propoxy oder für Dimethylamino.
- R³: steht bevorzugt für Wasserstoff, Hydroxy, Amino, für jeweils gegebenenfalls durch Fluor, Chlor, Cyano, Methoxy oder Ethoxy substituiertes Methyl, Ethyl, n- oder i-Propyl, n-, i-, s- oder t-Butyl, für jeweils gegebenenfalls durch Fluor, Chlor und/oder Brom substituiertes Ethenyl, Propenyl, Butenyl, Propinyl oder Butinyl, für jeweils gegebenenfalls durch Fluor, Chlor, Cyano, Methoxy oder Ethoxy substituiertes Methoxy, Ethoxy, n- oder i-Propoxy, n-, i-, s- oder t-Butoxy, Methylamino, Ethylamino, n- oder i-Propylamino, n-, i-, s- oder t-Butyl-amino, für Propenyloxy oder Butenyloxy, für Dimethylamino oder Diethyl-amino, für jeweils gegebenenfalls durch Fluor, Chlor, Methyl und/oder Ethyl substituiertes Cyclopropyl, Cyclobutyl, Cyclopentyl, Cyclohexyl, Cyclopropyl-amino, Cyclobutylamino, Cyclopentylamino, Cyclohexylamino, Cyclopropyl-methyl, Cyclobutylmethyl, Cyclopentylmethyl oder Cyclohexylmethyl, oder für jeweils gegebenenfalls durch Fluor, Chlor, Methyl, Trifluormethyl und/oder Methoxy substituiertes Phenyl oder Benzyl.
- R³: steht besonders bevorzugt für Methyl, Ethyl, n- oder i-Propyl, Methoxy, Ethoxy oder Cyclopropyl.
- R⁴: steht bevorzugt für Wasserstoff, Hydroxy, Mercapto, Amino, Cyano, Fluor, Chlor, Brom, für jeweils gegebenenfalls durch Fluor, Chlor, Cyano, Methoxy, Ethoxy, n- oder i-Propoxy, Acetyl, Propionyl, n- oder i-Buyroyl, Methoxy-carbonyl, Ethoxycarbonyl, n- oder i-Propoxy-carbonyl substituiertes Methyl, Ethyl, n- oder i-Propyl, n-, i-, s- oder t-Butyl, für jeweils gegebenenfalls durch Fluor, Chlor und/oder Brom substituiertes Ethenyl, Propenyl, Butenyl, Ethinyl, Propinyl oder Butinyl, für jeweils gegebenenfalls durch Fluor, Chlor, Cyano, Methoxy, Ethoxy, n- oder i-Propoxy, Methoxycarbonyl, Ethoxycarbonyl, n-oder i-Propoxy-carbonyl substituiertes Methoxy, Ethoxy, n- oder i-Propoxy, n-, i-, s- oder t-Butoxy, Methylthio, Ethylthio, n- oder i-Propylthio, n-, i-, s- oder t-Butylthio, Methylamino, Ethylamino, n- oder 1-Propylamino, n-, i-, s- oder t-Butylamino, Acetylamino oder Propionylamino, für Propenyloxy, Butenyloxy, Ethinyloxy, Propinyloxy, Butinyloxy, Propenylthio, Butenylthio, Propinylthio, Butinylthio, Propenylamino, Butenylamino, Propinylamino oder Butinylamino, für Dimethylamino, Diethylamino oder Dipropylamino, für jeweils gegebenenfalls durch Fluor, Chlor, Methyl und/oder Ethyl substituiertes Cyclopropyl, Cyclobutyl, Cyclopentyl, Cyclohexyl, Cyclopentenyl, Cyclohexenyl, Cyclo-propyloxy, Cyclobutyloxy, Cyclopentyloxy, Cyclohexyloxy, Cyclopropylthio, Cyclobutylthio, Cyclopentylthio, Cyclohexylthio, Cyclopropylamino, Cyclobutyl-amino, Cyclopentylamino, Cyclohexylamino, Cyclopropylmethyl, Cyclobutylmethyl, Cyclopentylmethyl, Cyclohexylmethyl, Cyclopropylmethoxy, Cyclobutylmethoxy, Cyclopentylmethoxy, Cyclohexylmethoxy, Cyclopropylmethylthio, Cyclobuiylmethylthio, Cyclopentylmethylthio, Cyclohexylmethylthio, Cyclopropylmethylamino, Cyclobutylmethylamino, Cyclopentylmethylamino oder Cyclohexylmethylamino, oder für jeweils gegebenenfalls durch Fluor, Chlor, Brom, Methyl, Trifluormethyl, Methoxy oder Methoxy-carbonyl substituiertes Phenyl, Benzyl, Phenoxy, Benzyloxy, Phenylthio, Benzylthio, Phenylamino oder Benzylamino.
- R⁴: steht besonders bevorzugt für ggf. durch Fluor substituiertes Methoxy, Ethoxy, n- oder i-Propoxy, n-, i-, s- oder t-Butoxy.
- R⁵, R⁶, R⁷ und R⁸: stehen bevorzugt unabhängig voneinander für Wasserstoff oder (C₁-C₃)-Alkyl.
- R⁵, R⁶, R⁷ und R⁸: stehen besonders bevorzugt unabhängig voneinander für Wasserstoff oder Methyl.
- R⁹: steht bevorzugt für Wasserstoff, Cyano, Fluor, Chlor, Brom, für jeweils gegebenenfalls durch Cyano, Fluor, Chlor, Methoxy oder Ethoxy substituiertes Methyl, Ethyl, n- oder i-Propyl, n-, i-, s- oder t-Butyl, Methoxy, Ethoxy, n- oder i-Propoxy, Methoxycarbonyl, Ethoxycarbonyl, n- oder 1-Propoxycarbonyl, , Methylthio, Ethylthio, n- oder 1-Propylthio, Methylsulfinyl, Ethylsulfinyl, Methylsulfonyl oder Ethylsulfonyl, oder für jeweils gegebenenfalls durch Cyano, Fluor oder Chlor substituiertes Propenyl, Butenyl, Propinyl, Butinyl, Propenyloxy, Butenyloxy, Propinyloxy oder Butinyloxy.
- R⁹: steht besonders bevorzugt für Wasserstoff, Fluor, Chlor, Brom, für jeweils gegebenenfalls durch Cyano, Fluor, Chlor, Methoxy oder Ethoxy substituiertes Methyl, Ethyl, n- oder i-Propyl, n-, i-, s- oder t-Butyl, Methoxy, Ethoxy, n- oder i-Propoxy.
- R⁹: steht am meisten bevorzugt für Wasserstoff, Methyl, Ethyl, n- oder i-Propyl.

Gegenstand der Erfindung sind vorzugsweise auch die Natrium-, Kalium-, Magnesium-, Calcium-, Ammonium-, (C₁-C₄)-Alkyl-ammonium-, Di-((C₁-C₄)-alkyl)-ammonium-, Tri-(C₁-C₄)-alkyl)-ammonium-, Tetra-((C₁-C₄)-alkyl)-ammonium-, Tri-((C₁-C₄)-alkyl)-sulfonium-, C₅- oder C₆-Cycloalkyl-ammonium- und Di-((C₁-C₂)-alkyl)-benzyl-ammonium-Salze von Verbindungen der Formel (I), in welcher A, Het, R, R¹, R², R³, R⁴, R⁵, R⁶, R⁷, R⁸ und R⁹ die oben vorzugsweise angegebene Bedeutung haben.

Die oben aufgeführten allgemeinen oder in Vorzugsbereichen angegebenen Restedefinitionen gelten sowohl für die Endprodukte der Formel (I) als auch entsprechend für die jeweils zu Herstellung benötigten Ausgangsstoffe bzw. Zwischenprodukte. Diese Restedefinitionen können untereinander, also auch zwischen den angegebenen Bereichen bevorzugter Verbindungen, beliebig kombiniert werden.

Erfindungsgemäß bevorzugt sind diejenigen Verbindungen der Formel (I), bei welchen eine Kombination der vorstehend als bevorzugt aufgeführten Bedeutungen vorliegt.

Erfindungsgemäß besonders bevorzugt sind diejenigen Verbindungen der Formel (I), bei welchen eine Kombination der vorstehend als besonders bevorzugt aufgeführten Bedeutungen vorliegt.

Die neuen substituierten Furylsulfonylaminocarbonylverbindungen der allgemeinen Formel (I) zeichnen sich durch starke herbizide Wirksamkeit aus.

Man erhält die neuen substituierten Furylsulfonylaminocarbonylverbindungen der allgemeinen Formel (I), wenn man
(a) substituierte Aminoazine der allgemeinen Formel (II) 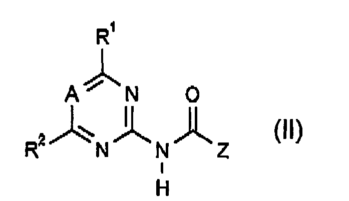 in welcher
   A, R¹ und R² die oben angegebene Bedeutung haben, und
   Z für Halogen, Alkoxy oder Aryloxy steht,
   mit Furanderivaten der allgemeinen Formel (III) 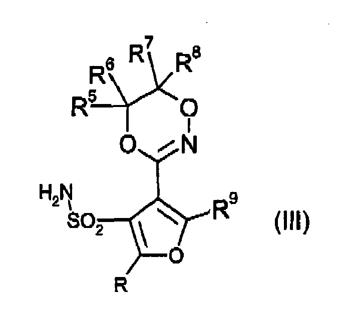 in welcher
   R und R⁵ bis R⁹ die oben angegebene Bedeutung haben,
   gegebenenfalls in Gegenwart eines Reaktionshilfsmittels und gegebenenfalls in Gegenwart eines Verdünnungsmittels umsetzt,
   oder (b) substituierte Furan-3-sulfonamide der allgemeinen Formel (III) 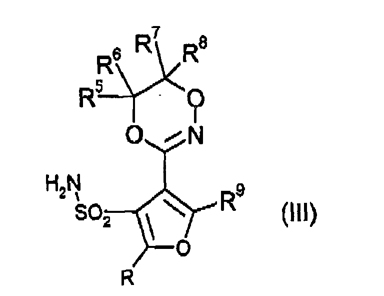 in welcher R, R⁹ und R⁵ bis R⁸ die oben angegebene Bedeutung haben,
   mit substituierten Triazolinonen der allgemeinen Formel (IV) 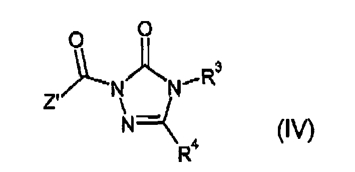 in welcher
   - R³ und R⁴: die oben angegebene Bedeutung haben und
   - Z': für Halogen, Alkoxy, Aryloxy oder Arylalkoxy steht,
   oder (c) substituierte Aminoazine der allgemeinen Formel (II) 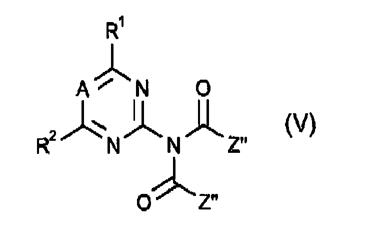 in welcher
   A, R¹ und R² die oben angegebene Bedeutung haben, und
   Z" für Halogen, Alkoxy oder Aryloxy steht,
   mit Furanderivaten der allgemeinen Formel (III) 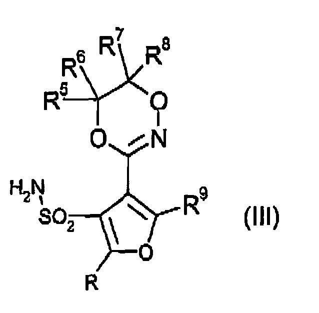 in welcher
   R und R⁵ bis R⁹ die oben angegebene Bedeutung haben,
   gegebenenfalls in Gegenwart eines Reaktionshilfsmittels und gegebenenfalls in Gegenwart eines Verdünnungsmittels umsetzt,
   oder (d) Aminoazine der allgemeinen Formel (VI) 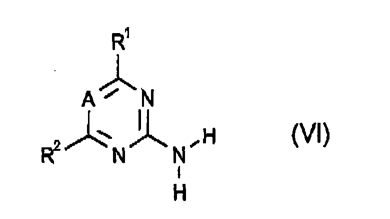 in welcher
   A, R¹ und R² die oben angegebene Bedeutung haben, und
   mit Furanderivaten der allgemeinen Formel (VII) 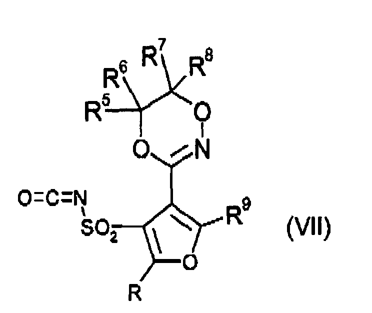 in welcher
   R und R⁵ bis R⁹ die oben angegebene Bedeutung haben,
   gegebenenfalls in Gegenwart eines Reaktionshilfsmittels und gegebenenfalls in Gegenwart eines Verdünnungsmittels umsetzt,
   und gegebenenfalls die nach den Verfahren (a) oder (b) erhaltenen Verbindungen der Formel (I) nach üblichen Methoden in Salze überführt.

Verwendet man beispielsweise 2-Methoxycarbonylamino-4-methoxy-6-ethyl-1,3,5-triazin und 2-Ethyl-4-(6,6-dihydro-[1,4,2]-dioxazin-3-yl)-furan-3-sulfonamid als Ausgangsstoffe, so kann der Reaktionsablauf beim erfindungsgemäßen Verfahren (a) durch das folgende Formelschema skizziert werden:

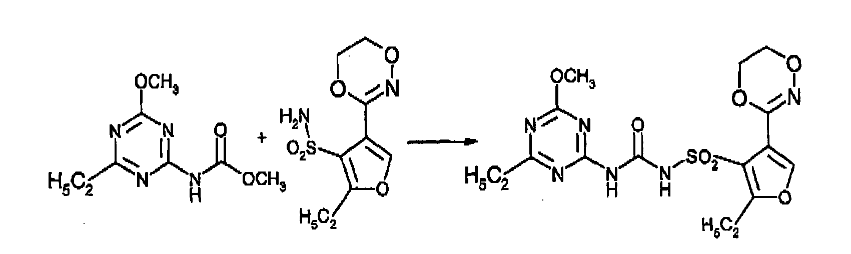

Die beim erfindungsgemäßen Verfahren zur Herstellung der Verbindungen der Formel (I) als Ausgangsstoffe zu verwendenden substituierten Aminoazine sind durch die Formel (II) allgemein definiert. In der Formel (II) haben A, R¹ und R² vorzugsweise bzw. insbesondere diejenigen Bedeutungen, die bereits oben im Zusammenhang mit der Beschreibung der Verbindungen der Formel (I) als bevorzugt bzw. als insbesondere bevorzugt für A, R¹ und R² angegeben wurden; Z steht vorzugsweise für Fluor, Chlor, Brom, (C₁-C₄)-Alkoxy oder Phenoxy, insbesondere für Chlor, Methoxy, Ethoxy oder Phenoxy.

Die Ausgangsstoffe der Formel (II) sind bekannt und/oder können nach an sich bekannten Verfahren hergestellt werden (vgl. US 4,690,707, DE 19501174).

Die beim erfindungsgemäßen Verfahren weiter als Ausgangsstoffe zu verwendenden Furanderivate sind durch die Formel (III) allgemein definiert. In der Formel (III) haben R und R⁹ und R⁶ bis R⁸ vorzugsweise bzw. insbesondere diejenigen Bedeutungen, die bereits oben im Zusammenhang mit der Beschreibung der Verbindungen der Formel (I) als bevorzugt bzw. als insbesondere bevorzugt für R bzw. R⁹ und R⁵ bis R⁸ angegeben wurden.

Die substituierten Furan-3-sulfonamide der allgemeinen Formel (III) sind noch nicht aus der Literatur bekannt, sie sind als solche ebenfalls Gegenstand der vorliegenden Erfindung. Unter den Verbindungen der Formel (III) sind solche bevorzugt, bei denen R für Wasserstoff, Methyl, Ethyl, n-Propyl und Isopropyl steht.

Man erhält die substituierten Furan-3-sulfonamide der allgemeinen Formel (III), wenn man substituierte Furan-3-sulfonamide der allgemeinen Formel (VIII) 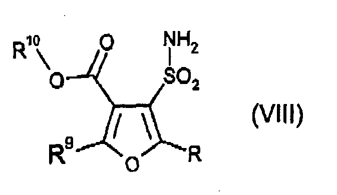 in welcher
R und R⁹die oben angegebene Bedeutung hat und R¹⁰ für (C₁-C₄)-Alkyl steht,
mit Hydroxylaminhydrochlorid und einem substituierten Alkan der Formel (IX) 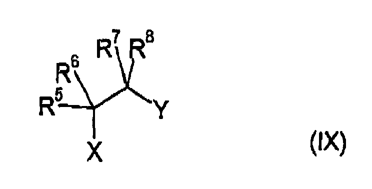 worin R⁵ bis R⁸ die oben angegebene Bedeutung haben und
X und Y unabhängig voneinander für Halogen, (C₁-C₆)-Alkylcarbonyloxy, (C₆-C₁₂)-Arylcarbonyloxy, (C₁-C₆)-Alkylsulfonyloxy oder (C₁-C₆)-Arylsulfonyloxy stehen,
gegebenenfalls in Gegenwart eines Verdünnungsmittels und gegebenenfalls in Gegenwart eines Reaktionshilfsmittels umsetzt (vgl. das Herstellungsbeispiel). Analoge Verfahren sind auch aus der US 5,476,936 und der WO 2006012982 bekannt.

Die Ausgangsstoffe der Formel (V) sind bekannt und/oder können nach an sich bekannten Verfahren hergestellt werden (vgl. die prioritätsältere noch nicht vorveröffentlichte DE-102006032164).

Die Alkane der Formel (VI) sind als Synthesechemikalien kommerziell erhältlich oder können nach an sich bekannten Verfahren hergestellt werden (vgl. US 5,476,936, Spalte 13).

Verwendet man beispielsweise 2,5-Dimethyl-4-(5,6-dihydro-[1,4,2]-dioxazin-3-yl)-furan-3-sulfonamid und 4,5-Dimethoxy-2-phenoxycarbonyl-2,4-dihydro-3H-1,2,4-triazol-3-on als Ausgangsstoffe, so kann der Reaktionsablauf beim erfindungsgemäßen Verfahren (b) durch das folgende Formelschema skizziert werden:

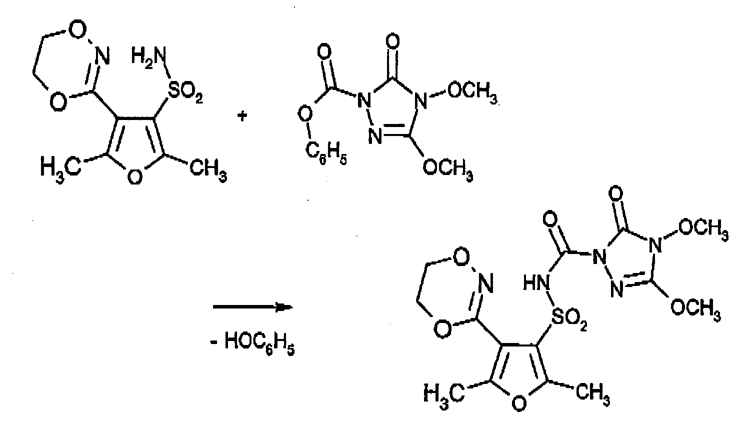

Die beim erfindungsgemäßen Verfahren (b) zur Herstellung von Verbindungen der allgemeinen Formel (I) als Ausgangsstoffe zu verwendenden substituierten Furan-3-sulfonamide sind durch die Formel (III) allgemein definiert. Es handelt sich um dieselben Verbindungen der Formel (III) die gemäß des erfindungsgemäßen Verfahrens (a) verwendet werden.

Die beim erfindungsgemäßen Verfahren (b) zur Herstellung von Verbindungen der allgemeinen Formel (I) weiter als Ausgangsstoffe zu verwendenden substituierten Triazolinone sind durch die Formel (IV) allgemein definiert. In der allgemeinen Formel (IV) haben R³ und R⁴ vorzugsweise bzw. insbesondere diejenigen Bedeutungen, die bereits oben im Zusammenhang mit der Beschreibung der erfindungsgemäßen Verbindungen der allgemeinen Formel (I) vorzugsweise bzw. als insbesondere bevorzugt für R³ und R³ angegeben worden sind.

Die Ausgangsstoffe der allgemeinen Formel (IV) sind bekannt und/oder können nach an sich bekannten Verfahren hergestellt werden (vgl. auch Hinweise in WO 01/05788). Z' in der Formel (IV) steht bevorzugt für Chlor, Brom, Methoxy, Ethoxy, Phenoxy oder Benzyloxy.

Als Verdünnungsmittel zur Durchführung der erfindungsgemäßen Verfahren (a) und (b) und des Verfahrens zur Herstellung derZwischenprodukte der Formel (III) kommen vor allem inerte organische Lösungsmittel in Betracht. Hierzu gehören insbesondere aliphatische, alicyclische oder aromatische, gegebenenfalls halogenierte Kohlenwasserstoffe, wie beispielsweise Benzin, Benzol, Toluol, Xylol, Chlorbenzol, Dichlorbenzol, Petrolether, Hexan, Cyclohexan, Dichlormethan, Chloroform, Tetrachlorkohlenstoff; Ether, wie Diethylether, Diisopropylether, Dioxan, Tetrahydrofuran oder Ethylenglykoldimethyl- oder -diethylether; Ketone, wie Aceton, Butanon oder Methyl-isobutyl-keton; Nitrile, wie Acetonitril, Propionitril oder Benzonitril; Amide, wie N,N-Dimethylformamid, N,N-Dimethylacetamid, N-Methyl-formanilid, N-Methylpyrrolidon oder Hexamethylphosphorsäuretriamid; Ester wie Essigsäuremethylester oder Essigsäureethylester, sowie Sulfoxide, wie Dimethylsulfoxid.

Die erfindungsgemäßen Verfahren (a), (b), (c) und (d) sowie das Verfahren zur Herstellung der Zwischenprodukte der Formel (III) werden vorzugsweise in Gegenwart eines geeigneten Reaktionshilfsmittels durchgeführt. Als solche kommen alle üblichen anorganischen oder organischen Basen infrage. Hierzu gehören beispielsweise Erdalkali- oder Alkalimetall- -hydride, -hydroxide, -amide, -alkoholate, - acetate, -carbonate oder -hydrogencarbonate, wie beispielsweise Natriumhydrid, Natriumamid, Natriummethylat, Natriumethylat, Kalium-tert.-butylat, Natriumhydroxid, Kaliumhydroxid, Ammoniumhydroxid, Natriumacetat, Kaliumacetat, Calciumacetat, Ammoniumacetat, Natriumcarbonat, Kaliumcarbonat, Kaliumhydrogencarbonat, Natriumhydrogencarbonat oder Ammoniumcarbonat sowie tertiäre Amine, wie Trimethylamin, Triethylamin, Tributylamin, N,N-Dimethylanilin, Pyridin, N-Methylpiperidin, N,N-Dimethylaminopyridin, Diazabicyclooctan (DABCO), Diazabicyclononen (DBN) oder Diazabicycloundecen (DBU).

Die Reaktionstemperaturen können bei der Durchführung des erfindungsgemäßen Verfahrens (a) in einem größeren Bereich variiert werden. Im Allgemeinen arbeitet man bei Temperaturen zwischen -20°C und +150°C, vorzugsweise zwischen -10°C und +120°C.

Die Reaktionstemperaturen können bei der Durchführung des erfindungsgemäßen Verfahrens (b) in einem größeren Bereich variiert werden. Im Allgemeinen arbeitet man bei Temperaturen zwischen -20°C und +150°C, vorzugsweise zwischen 0°C und +100°C.

Die Reaktionstemperaturen können bei der Durchführung des erfindungsgemäßen Verfahrens zur Herstellung der Zwischenprodukte der Formel (III) in einem größeren Bereich variiert werden. Im Allgemeinen arbeitet man bei Temperaturen zwischen 0°C und +150°C, vorzugsweise zwischen 10°C und +80°C.

Die erfindungsgemäßen Verfahren (a) und (b) und das Verfahren zur Herstellung Zwischenprodukte der Formel (III) werden im Allgemeinen unter Normaldruck durchgeführt. Es ist jedoch auch möglich, die erfindungsgemäßen Verfahren unter erhöhtem oder vermindertem Druck - im Allgemeinen zwischen 0,1 bar und 10 bardurchzuführen.

Zur Durchführung der erfindungsgemäßen Verfahren (a) und (b) und des Verfahrens zur Herstellung Zwischenprodukte der Formel (III) werden die Ausgangsstoffe im Allgemeinen in angenähert äquimolaren Mengen eingesetzt. Es ist jedoch auch möglich, eine der Komponenten in einem größeren Überschuss zu verwenden. Die Umsetzung wird im Allgemeinen in einem geeigneten Verdünnungsmittel in Gegenwart eines Reaktionshilfsmittels durchgeführt und das Reaktionsgemisch wird im Allgemeinen mehrere Stunden bei der erforderlichen Temperatur gerührt. Die Aufarbeitung wird nach üblichen Methoden durchgeführt (vgl. die Herstellungsbeispiele).

Aus den erfindungsgemäßen Verbindungen der allgemeinen Formel (I) können gegebenenfalls Salze hergestellt werden. Man erhält solche Salze in einfacher Weise nach üblichen Salzbildungsmethoden, beispielsweise durch Lösen oder Dispergieren einer Verbindung der Formel (I) in einem geeigneten Lösungsmittel, wie z.B. Methylenchlorid, Aceton, tert-Butyl-methylether, Wasser oder Toluol, und Zugabe einer geeigneten Base. Die Salze können dann - gegebenenfalls nach längerem Rühren - durch Einengen oder Absaugen isoliert werden.

Die erfindungsgemäßen Wirkstoffe können als Defoliants, Desiccants, Krautabtötungsmittel und insbesondere als Unkrautvernichtungsmittel verwendet werden. Unter Unkraut im weitesten Sinne sind alle Pflanzen zu verstehen, die an Orten aufwachsen, wo sie unerwünscht sind. Ob die erfindungsgemäßen Stoffe als totale oder selektive Herbizide wirken, hängt im wesentlichen von der angewendeten Menge ab.

Die erfindungsgemäßen Verbindungen der Formel (I) und deren Salze, im folgenden zusammen als (erfindungsgemäße) Verbindungen der Formel (I) bezeichnet, weisen eine ausgezeichnete herbizide Wirksamkeit gegen ein breites Spektrum wirtschaftlich wichtiger mono- und dikotyler Schadpflanzen auf. Auch schwer bekämpfbare perennierende Unkräuter, die aus Rhizomen, Wurzelstöcken oder anderen Dauerorganen austreiben, werden durch die Wirkstoffe gut erfaßt. Dabei ist es gleichgültig, ob die Substanzen im Vorsaat-, Vorauflauf- oder Nachauflaufverfahren ausgebracht werden.
Werden die erfindungsgemäßen Verbindungen vor dem Keimen auf die Erdoberfläche appliziert, so wird entweder das Auflaufen der Unkrautkeimlinge vollständig verhindert oder die Unkräuter wachsen bis zum Keimblattstadium heran, stellen jedoch dann ihr Wachstum ein und sterben schließlich nach Ablauf von drei bis vier Wochen vollkommen ab.
Bei Applikation der Wirkstoffe auf die grünen Pflanzenteile im Nachauflaufverfahren tritt ebenfalls sehr rasch nach der Behandlung ein drastischer Wachstumsstop ein, und die Unkrautpflanzen bleiben in dem zum Applikationszeitpunkt vorhandenen Wachstumsstadium stehen oder sterben nach einer gewissen Zeit ganz ab, so daß auf diese Weise eine für die Kulturpflanzen schädliche Unkrautkonkurrenz sehr früh und nachhaltig beseitigt wird.
Obgleich die erfindungsgemäßen Verbindungen eine ausgezeichnete herbizide Aktivität gegenüber mono- und dikotylen Unkräutern aufweisen, werden Kulturpflanzen wirtschaftlich bedeutender Kulturen wie z.B. Weizen, Gerste, Roggen, Reis, Mais, Zuckerrübe, Baumwolle und Soja nur unwesentlich oder gar nicht geschädigt. Die vorliegenden Verbindungen eignen sich aus diesen Gründen sehr gut zur selektiven Bekämpfung von unerwünschtem Pflanzenwuchs in landwirtschaftlichen Nutzpflanzungen.
Darüberhinaus weisen die erfindungsgemäßen Substanzen hervorragende wachstumsregulatorische Eigenschaften bei Kulturpflanzen auf. Sie greifen regulierend in den pflanzeneigenen Stoffwechsel ein und können damit zur gezielten Beeinflussung von Pflanzeninhaltsstoffen und zur Emteerleichterung wie z.B. durch Auslösen von Desikkation und Wuchsstauchung eingesetzt werden. Desweiteren eignen sie sich auch zur generellen Steuerung und Hemmung von unerwünschtem vegetativen Wachstum, ohne dabei die Pflanzen abzutöten. Eine Hemmung des vegetativen Wachstums spielt bei vielen mono- und dikotylen Kulturen eine große Rolle, da das Lagern hierdurch verringert oder völlig verhindert werden kann. Aufgrund ihrer herbiziden und pflanzenwachstumsregulatorischen Eigenschaften können die Wirkstoffe auch zur Bekämpfung von Schadpflanzen in Kulturen von bekannten oder noch zu entwickelnden gentechnisch veränderten Pflanzen eingesetzt werden. Die transgenen Pflanzen zeichnen sich in der Regel durch besondere vorteilhafte Eigenschaften aus, beispielsweise durch Resistenzen gegenüber bestimmten Pestiziden, vor allem bestimmter Herbizide, Resistenzen gegenüber Pflanzenkrankheiten oder Erregern von Pflanzenkrankheiten wie bestimmten Insekten oder Mikroorganismen wie Pilzen, Bakterien oder Viren. Andere besondere Eigenschaften betreffen z. B. das Erntegut hinsichtlich Menge, Qualität, Lagerfähigkeit, Zusammensetzung und spezieller Inhaltsstoffe. So sind transgene Pflanzen mit erhöhtem Stärkegehalt oder veränderter Qualität der Stärke oder solche mit anderer Fettsäurezusammensetzung des Emteguts bekannt. Bevorzugt ist die Anwendung der erfindungsgemäßen Verbindungen der Formel (I) oder deren Salze in wirtschaftlich bedeutenden transgenen Kulturen von Nutz- und Zierpflanzen, z. B. von Getreide wie Weizen, Gerste, Roggen, Hafer, Hirse, Reis, Maniok und Mais oder auch Kulturen von Zuckerrübe, Baumwolle, Soja, Raps, Kartoffel, Tomate, Erbse und anderen Gemüsesorten.
Vorzugsweise können die Verbindungen der Formel (I) als Herbizide in Nutzpflanzenkulturen eingesetzt werden, welche gegenüber den phytotoxischen Wirkungen der Herbizide resistent sind bzw. gentechnisch resistent gemacht worden sind.

Herkömmliche Wege zur Herstellung neuer Pflanzen, die im Vergleich zu bisher vorkommenden Pflanzen modifizierte Eigenschaften aufweisen, bestehen beispielsweise in klassischen Züchtungsverfahren und der Erzeugung von Mutanten. Alternativ können neue Pflanzen mit veränderten Eigenschaften mit Hilfe gentechnischer Verfahren erzeugt werden (siehe z. B. EP-A-0221044, EP-A-0131624). Beschrieben wurden beispielsweise in mehreren Fällen
- gentechnische Veränderungen von Kulturpflanzen zwecks Modifikation der in den Pflanzen synthetisierten Stärke (z. B. WO 92/11376, WO 92/14827, WO 91/19806),
- transgene Kulturpflanzen, welche gegen bestimmte Herbizide vom Typ Glufosinate (vgl. z. B. EP-A-0242236, EP-A-242246) oder Glyphosate (WO 92/00377) oder der Sulfonylharnstoffe (EP-A-0257993, US-A-5013659) resistent sind,
- transgene Kulturpflanzen, beispielsweise Baumwolle, mit der Fähigkeit Bacillus thuringiensis-Toxine (Bt-Toxine) zu produzieren, welche die Pflanzen gegen bestimmte Schädlinge resistent machen (EP-A-0142924, EP-A-0193259).
- transgene Kulturpflanzen mit modifizierter Fettsäurezusammensetzung (WO 91/13972).

Zahlreiche molekularbiologische Techniken, mit denen neue transgene Pflanzen mit veränderten Eigenschaften hergestellt werden können, sind im Prinzip bekannt; siehe z.B. Sambrook et al., 1989, Molecular Cloning, A Laboratory Manual, 2. Aufl. Cold Spring Harbor Laboratory Press, Cold Spring Harbor, NY; oder Winnacker "Gene und Klone", VCH Weinheim 2. Auflage 1996 oder Christou, "Trends in Plant Science" 1 (1996) 423-431).
Für derartige gentechnische Manipulationen können Nucleinsäuremoleküle in Plasmide eingebracht werden, die eine Mutagenese oder eine Sequenzveränderung durch Rekombination von DNA-Sequenzen erlauben. Mit Hilfe der obengenannten Standardverfahren können z. B. Basenaustausche vorgenommen, Teilsequenzen entfernt oder natürliche oder synthetische Sequenzen hinzugefügt werden. Für die Verbindung der DNA-Fragmente untereinander können an die Fragmente Adaptoren oder Linker angesetzt werden.
Die Herstellung von Pflanzenzellen mit einer verringerten Aktivität eines Genprodukts kann beispielsweise erzielt werden durch die Expression mindestens einer entsprechenden antisense-RNA, einer sense-RNA zur Erzielung eines Cosuppressionseffektes oder die Expression mindestens eines entsprechend konstruierten Ribozyms, das spezifisch Transkripte des obengenannten Genprodukts spaltet.
Hierzu können zum einen DNA-Moleküle verwendet werden, die die gesamte codierende Sequenz eines Genprodukts einschließlich eventuell vorhandener flankierender Sequenzen umfassen, als auch DNA-Moleküle, die nur Teile der codierenden Sequenz umfassen, wobei diese Teile lang genug sein müssen, um in den Zellen einen antisense-Effekt zu bewirken. Möglich ist auch die Verwendung von DNA-Sequenzen, die einen hohen Grad an Homologie zu den codiereden Sequenzen eines Genprodukts aufweisen, aber nicht vollkommen identisch sind.
Bei der Expression von Nucleinsäuremolekülen in Pflanzen kann das synthetisierte Protein in jedem beliebigen Kompartiment der pflanzlichen Zelle lokalisiert sein. Um aber die Lokalisation in einem bestimmten Kompartiment zu erreichen, kann z. B. die codierende Region mit DNA-Sequenzen verknüpft werden, die die Lokalisierung in einem bestimmten Kompartiment gewährleisten. Derartige Sequenzen sind dem Fachmann bekannt (siehe beispielsweise Braun et al., EMBO J. 11 (1992), 3219-3227; Wolter et al., Proc. Natl. Acad. Sci. USA 85 (1988), 846-850; Sonnewald et al., Plant J. 1 (1991), 95-106).
Die transgenen Pflanzenzellen können nach bekannten Techniken zu ganzen Pflanzen regeneriert werden. Bei den transgenen Pflanzen kann es sich prinzipiell um Pflanzen jeder beliebigen Pflanzenspezies handeln, d.h., sowohl monokotyle als auch dikotyle Pflanzen.
So sind transgene Pflanzen erhältlich, die veränderte Eigenschaften durch Überexpression, Suppression oder Inhibierung homologer (= natürlicher) Gene oder Gensequenzen oder Expression heterologer (= fremder) Gene oder Gensequenzen aufweisen.
Vorzugsweise können die erfindungsgemäßen Verbindungen (I) in transgenen Kulturen eingesetzt werden, welche gegen Herbizide aus der Gruppe der Sulfonylharnstoffe, Glufosinate-ammonium oder Glyphosate-isopropylammonium und analoge Wirkstoffe resistent sind.
Bei der Anwendung der erfindungsgemäßen Wirkstoffe in transgenen Kulturen treten neben den in anderen Kulturen zu beobachtenden Wirkungen gegenüber Schadpflanzen oftmals Wirkungen auf, die für die Applikation in der jeweiligen transgenen Kultur spezifisch sind, beispielsweise ein verändertes oder speziell erweitertes Unkrautspektrum, das bekämpft werden kann, veränderte Aufwandmengen, die für die Applikation eingesetzt werden können, vorzugsweise gute Kombinierbarkeit mit den Herbiziden, gegenüber denen die transgene Kultur resistent ist, sowie Beeinflussung von Wuchs und Ertrag der transgenen Kulturpflanzen.
Gegenstand der Erfindung ist deshalb auch die Verwendung der erfindungsgemäßen Verbindungen der Formel (I) als Herbizide zur Bekämpfung von Schadpflanzen in transgenen Kulturpflanzen.
Die erfindungsgemäßen Verbindungen können in Form von Spritzpulvern, emulgierbaren Konzentraten, versprühbaren Lösungen, Stäubemitteln oder Granulaten in den üblichen Zubereitungen angewendet werden. Gegenstand der Erfindung sind deshalb auch herbizide und pflanzenwachstumsregulierende Mittel, die die Verbindungen der Formel (I) enthalten.
Die Verbindungen der Formel (I) können auf verschiedene Art formuliert werden, je nachdem welche biologischen und/oder chemisch-physikalischen Parameter vorgegeben sind. Als Formulierungsmöglichkeiten kommen beispielsweise in Frage: Spritzpulver (WP), wasserlösliche Pulver (SP), wasserlösliche Konzentrate, emulgierbare Konzentrate (EC), Emulsionen (EW), wie Öl-in-Wasser- und Wasserin-Öl-Emulsionen, versprühbare Lösungen, Suspensionskonzentrate (SC). Dispersionen auf Öl- oder Wasserbasis, ölmischbare Lösungen, Kapselsuspensionen (CS), Stäubemittel (DP), Beizmittel, Granulate für die Streu- und Bodenapplikation, Granulate (GR) in Form von Mikro-, Sprüh-, Aufzugs- und Adsorptionsgranulaten, wasserdispergierbare Granulate (WG), wasserlösliche Granulate (SG), ULV-Formulierungen, Mikrokapseln und Wachse.

Diese einzelnen Formulierungstypen sind im Prinzip bekannt und werden beispielsweise beschrieben in: Winnacker-Küchler, "Chemische Technologie", Band 7, C. Hanser Verlag München, 4. Aufl. 1986, Wade van Valkenburg, "Pesticide Formulations", Marcel Dekker, N.Y., 1973; K. Martens, "Spray Drying" Handbook, 3rd Ed. 1979, G. Goodwin Ltd. London.
Die notwendigen Formulierungshilfsmittel wie Inertmaterialien, Tenside, Lösungsmittel und weitere Zusatzstoffe sind ebenfalls bekannt und werden beispielsweise beschrieben in: Watkins, "Handbook of Insecticide Dust Diluents and Carriers", 2nd Ed., Darland Books, Caldwell N.J., H.v. Olphen, "Introduction to Clay Colloid Chemistry"; 2nd Ed., J. Wiley & Sons, N.Y.; C. Marsden, "Solvents Guide"; 2nd Ed., Interscience, N.Y. 1963; McCutcheon's "Detergents and Ernulsifiers Annual", MC Publ. Corp., Ridgewood N.J.; Sisley and Wood, "Encyclopedia of Surface Active Agents", Chem. Publ. Co. Inc., N.Y. 1964; Schönfeldt, "Grenzflächenaktive Äthylenoxidaddukte", Wiss. Verlagsgesell., Stuttgart 1976; Winnacker-Küchler, "Chemische Technologie", Band 7, C. Hanser Verlag München, 4. Aufl. 1986.
Auf der Basis dieser Formulierungen lassen sich auch Kombinationen mit anderen pestizid wirksamen Stoffen, wie z.B. Insektiziden, Akariziden, Herbiziden, Fungiziden, sowie mit Safenem, Düngemitteln und/oder Wachstumsregulatoren herstellen, z.B. in Form einer Fertigformulierung oder als Tankmix. Spritzpulver sind in Wasser gleichmäßig dispergierbare Präparate, die neben dem Wirkstoff außer einem Verdünnungs- oder Inertstoff noch Tenside ionischer und/oder nichtionischer Art (Netzmittel, Dispergiermittel), z.B. polyoxyethylierte Alkylphenole, polyoxethylierte Fettalkohole, polyoxethylierte Fettamine, Fettalkoholpolyglykolethersulfate, Alkansulfonate, Alkylbenzolsulfonate, ligninsulfonsaures Natrium, 2,2'-dinaphthylmethan-6,6'-disulfonsaures Natrium, dibutylnaphthalin-sulfonsaures Natrium oder auch oleoylmethyltaurinsaures Natrium enthalten. Zur Herstellung der Spritzpulver werden die herbiziden Wirkstoffe beispielsweise in üblichen Apparaturen wie Hammermühlen, Gebläsemühlen und Luftstrahlmühlen feingemahlen und gleichzeitig oder anschließend mit den Formulierungshilfsmitteln vermischt.

Emulgierbare Konzentrate werden durch Auflösen des Wirkstoffes in einem organischen Lösungsmittel z.B. Butanol, Cyclohexanon, Dimethylformamid, Xylol oder auch höhersiedenden Aromaten oder Kohlenwasserstoffen oder Mischungen der organischen Lösungsmittel unter Zusatz von einem oder mehreren Tensiden ionischer und/oder nichtionischer Art (Emulgatoren) hergestellt. Als Emulgatoren können beispielsweise verwendet werden: Alkylarylsulfonsaure Calcium-Salze wie Ca-dodecylbenzolsulfonat oder nichtionische Emulgatoren wie Fettsäurepolyglykolester, Alkylarylpolyglykolether, Fettalkoholpolyglykolether, Propylenoxid-Ethylenoxid-Kondensationsprodukte, Alkylpolyether, Sorbitan-ester wie z.B. Sorbitanfettsäureester oder Polyoxethylensorbitanester wie z.B. Polyoxyethylensorbitanfettsäureester.
Stäubemittel erhält man durch Vermahlen des Wirkstoffes mit fein verteilten festen Stoffen, z.B. Talkum, natürlichen Tonen, wie Kaolin, Bentonit und Pyrophyllit, oder Diatomeenerde.
Suspensionskonzentrate können auf Wasser- oder Ölbasis sein. Sie können beispielsweise durch Naß-Vermahlung mittels handelsüblicher Perlmühlen und gegebenenfalls Zusatz von Tensiden, wie sie z.B. oben bei den anderen Formulierungstypen bereits aufgeführt sind, hergestellt werden.
Emulsionen, z.B. Öl-in-Wasser-Emulsionen (EW), lassen sich beispielsweise mittels Rührern, Kolloidmühlen und/oder statischen Mischern unter Verwendung von wäßrigen organischen Lösungsmitteln und gegebenenfalls Tensiden, wie sie z.B. oben bei den anderen Formulierungstypen bereits aufgeführt sind, herstellen. Granulate können entweder durch Verdüsen des Wirkstoffes auf adsorptionsfähiges, granuliertes Inertmaterial hergestellt werden oder durch Aufbringen von Wirkstoffkonzentraten mittels Klebemitteln, z.B. Polyvinylalkohol, polyacrylsaurem Natrium oder auch Mineralölen, auf die Oberfläche von Trägerstoffen wie Sand, Kaolinite oder von granuliertem Inertmaterial. Auch können geeignete Wirkstoffe in der für die Herstellung von Düngemittelgranulaten üblichen Weise - gewünschtenfalls in Mischung mit Düngemitteln - granuliert werden. Wasserdispergierbare Granulate werden in der Regel nach den üblichen Verfahren wie Sprühtrocknung, Wirbelbett-Granulierung, Teller-Granulierung, Mischung mit Hochgeschwindigkeitsmischern und Extrusion ohne festes Inertmaterial hergestellt.

Zur Herstellung von Teller-, Fließbett-, Extruder- und Sprühgranulate siehe z.B. Verfahren in "Spray-Drying Handbook" 3rd ed. 1979, G. Goodwin Ltd., London; J.E. Browning, "Agglomeration", Chemical and Engineering 1967, Seiten 147 ff; "Perry's Chemical Engineer's Handbook", 5th Ed., McGraw-Hill, New York 1973, S. 8-57.
Für weitere Einzelheiten zur Formulierung von Pflanzenschutzmitteln siehe z.B. G.C. Klingman, "Weed Control as a Science", John Wiley and Sons, Inc., New York, 1961, Seiten 81-96 und J.D. Freyer, S.A. Evans, "Weed Control Handbook", 5th Ed., Blackwell Scientific Publications, Oxford, 1968, Seiten 101-103.
Die agrochemischen Zubereitungen enthalten in der Regel 0,1 bis 99 Gew.-%, vorzugsweise 0,1 bis 95 Gew.-%, insbesondere bevorzugt 0.5 bis 90 Gew.-% Wirkstoff der Formel (I).
In Spritzpulvern beträgt die Wirkstoffkonzentration z.B. etwa 10 bis 90 Gew.-%, der Rest zu 100 Gew.-% besteht aus üblichen Formulierungsbestandtellen. Bei emulgierbaren Konzentraten kann die Wirkstoffkonzentration etwa 1 bis 90, vorzugsweise 5 bis 80 Gew.-% betragen. Staubförmige Formulierungen enthalten 1 bis 30 Gew.-% Wirkstoff, vorzugsweise meistens 5 bis 20 Gew.-% an Wirkstoff, versprühbare Lösungen enthalten etwa 0,05 bis 80, vorzugsweise 2 bis 50 Gew.-% Wirkstoff. Bei wasserdispergierbaren Granulaten hängt der Wirkstoffgehalt zum Teil davon ab, ob die wirksame Verbindung flüssig oder fest vorliegt und welche Granulierhilfsmittel, Füllstoffe usw. verwendet werden. Bei den in Wasser dispergierbaren Granulaten liegt der Gehalt an Wirkstoff beispielsweise zwischen 1 und 95 Gew.-%, vorzugsweise zwischen 10 und 80 Gew.-% .
Daneben enthalten die genannten Wirkstofformulierungen gegebenenfalls die jeweils üblichen Haft-, Netz-, Dispergier-, Emulgier-, Penetrations-, Konservierungs-, Frostschutz- und Lösungsmittel, Füll-, Träger- und Farbstoffe, Entschäumer, Verdunstungshemmer und den pH-Wert und die Viskosität beeinflussende Mittel.
Im Falle der Benutzung von Wasser als Streckmittel können z.B. auch organische Lösungsmittel als Hilfslösungsmittel verwendet werden. Als flüssige Lösungsmittel kommen im wesentlichen in Frage: Aromaten, wie Xylol, Toluol, oder Alkylnaphthaline, chlorierte Aromaten und chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdöffraktionen, mineralische und pflanzliche Öle, Alkohole, wie Butanol oder Glykol sowie deren Ether und Ester, Ketone wie Aceton, Methylethylketon, Methylisobutylketon oder Cyclohexanon, stark polare Lösungsmittel, wie Dimethylformamid und Dimethylsulfoxid, sowie Wasser.

Als feste Trägerstoffe kommen in Frage: z.B. Ammoniumsalze und natürliche Gesteinsmehle, wie kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate, als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengeln; als Emulgier- und/oder schaumerzeugende Mittel kommen in Frage: z.B. nichtionogene und anionische Emulgatoren, wie Polyoxyethylen-Fettsäure-Ester, Polyoxyethylen-Fettalkohol-Ether, z.B. Alkylarylpolyglykolether, Alkylsulfonate, Alkylsulfate, Arylsulfonate sowie Eiweißhydrolysate; als Dispergiermittel kommen in Frage: z.B. Lignin-Sulfitablaugen und Methylcellulose.

Es können in den Formulierungen Haftmittel wie Carboxymethylcellulose, natürliche und synthetische pulvrige, körnige oder latexförmige Polymere verwendet werden, wie Gummiarabicum, Polyvinylalkohol, Polyvinylacetat, sowie natürliche Phospholipide, wie Kephaline und Lecithine und synthetische Phospholipide. Weitere Additive können mineralische und vegetabile Öle sein.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe wie Salze von Eisen, Mangan, Bor, Kupfer, Kobalt, Molybdän und Zink verwendet werden.

Die Verbindungen der Formel (I) oder deren Salze können als solche oder in Form ihrer Zubereitungen (Formulierungen) mit anderen pestizid wirksamen Stoffen, wie z. B. Insektiziden, Akariziden, Nematiziden, Herbiziden, Fungiziden, Safenern, Düngemitteln und/oder Wachstumsregulatoren kombiniert eingesetzt werden, z. B. als Fertigformulierung oder als Tankmischungen. Als Kombinationspartner für die erfindungsgemäßen Wirkstoffe in Mischformulierungen oder im Tank-Mix sind beispielsweise bekannte Wirkstoffe, die auf einer Inhibition von beispielsweise Acetolactat-Synthase, Acetyl-Coenzyl-A-Carboxylase, PS I, PS II, HPPDO, Phytoene-Desaturase, Protoporphyrinogen-Oxidase, Glutamine-Synthetase, Cellulosebiosynthese, 5-Enolpyruvylshikimat-3-phosphat-Synthetase beruhen, einsetzbar. Solche Verbindungen und auch andere einsetzbare Verbindungen mit teilweise unbekanntem oder anderem Wirkungsmechanismus sind z.B. in Weed Research 26, 441-445 (1986), oder "The Pesticide Manual", 11. Auflage 1997 (im Folgenden auch kurz "PM") und 12. Auflage 2000, The British Crop Protection Council and the Royal Soc. of Chemistry (Herausgeber), und dort zitierter Literatur beschrieben. Als literaturbekannte Herbizide, die mit den Verbindungen der Formel (I) kombiniert werden können, sind z.B. folgende Wirkstoffe zu nennen (Anmerkung: Die Verbindungen sind entweder mit dem "common name" nach der International Organization for Standardization (ISO) oder mit dem chemischen Namen, ggf. zusammen mit einer üblichen Codenummer bezeichnet): acetochlor; acifluorfen(-sodium); aclonifen; AKH 7088, d.h. [[[1-[5-[2-Chloro-4-(trifluoromethyl)-phenoxy]-2-nitrophenyl]-2-methoxyethylidene]-amino]-oxy]-essigsäure und -essigsäuremethylester; alachlor; alloxydim(-sodium); ametryn; amicarbazone, amidochlor, amidosulfuron; aminopyralid; amitrol; AMS, d.h. Ammoniumsulfamat; anilofos; asulam; atrazin; azafenidin, azimsulfuron (DPX-A8947); aziprotryn; barban; BAS 516 H, d.h. 5-Fluor-2-phenyl-4H-3,'1-benzoxazin-4-on; beflubutamid, benazolin(-ethyl); bencarbazone; benfluralin; benfuresate; bensulfuron(-methyl); bensulide; bentazone; benzobicyclon, benzofenap; benzofluor; benzoylprop(-ethyl); benzthiazuron; bialaphos; bifenox; bispyribac(-sodium), bromacil; bromobutide; bromofenoxim; bromoxynil; bromuron; buminafos; busoxinone; butachlor; butafenacil, butamifos; butenachlor; buthidazole; butralin; butroxydim, butylate; cafenstrole (CH-900); carbetamide; carfentrazone(-ethyl); caloxydim, CDAA, d.h. 2-Chlor-N,N-di-2-propenylacetamid; CDEC, d.h. Diethyldithiocarbaminsäure-2-chlorallylester; chlomethoxyfen; chloramben; chlorazifop-butyl, chlormesulon; chlorbromuron; chlorbufam; chlorfenac; chlorflurecol-methyl; chloridazon; chlorimuron(-ethyl); chlornitrofen; chlorotoluron; chloroxuron; chlorpropham; chlorsulfuron; chlorthal-dimethyl; chlorthiamid; chlortoluron, cinidon(-methyl und -ethyl), cinmethylin; cinosulfuron; clefoxydim, clethodim; clodinafop und dessen Esterderivate (z.B. clodinafop-propargyl); clomazone; clomeprop; cloproxydim; clopyralid; clopyrasulfuron(-methyl), cloransulam(-methyl), cumyluron (JC 940); cyanazine; cycloate; cyclosulfamuron (AC 104); cycloxydim; cycluron; cyhalofop und dessen Esterderivate (z.B. Butylester, DEH-112); cyperquat; cyprazine; cyprazole; daimuron; 2,4-D, 2,4-DB, dalapon; desmedipham; desmetryn; di-allate; dicamba; dichlobenil; dichlorprop; diclofop und dessen Ester wie diclofop-methyl; diclosulam, diethatyl(-ethyl); difenoxuron; difenzoquat; diflufenican; diflufenzopyr, dimefuron; dimepiperate, dimethachlor; dimethametryn; dimethenamid (SAN-582H); dimethazone, dimexyflam, dimethipin; dimetrasulfuron, dinitramine; dinoseb; dinoterb; diphenamid; dipropetryn; diquat; dithiopyr; diuron; DNOC; eglinazine-ethyl; EL 77, d.h. 5-Cyano-1-(1,1-dimethylethyl)-N-methyl-1H-pyrazole-4-carboxamid; endothal; epoprodan, EPTC; esprocarb; ethalfluralin; ethametsulfuron-methyl; ethidimuron; ethiozin; ethofumesate; ethoxyfen und dessen Ester (z.B. Ethylester, HN-252); ethoxysulfuron, etobenzanid (HW 52); F5231, d.h. N-[2-Chlor-4-fluor-5-[4-(3-fluorpropyl)-4,5-dihydro-5-oxo-1H-tetrazol-1-yl]-phenyl]-ethansulfonamid; fenoprop; fenoxan, fenoxaprop und fenoxaprop-P sowie deren Ester, z.B. fenoxaprop-P-ethyl und fenoxaprop-ethyl; fenoxydim; fentrazamide, fenuron; flamprop(-methyl oder -isopropyl oder -isopropyl-L); flazasulfuron; floazulate, florasulam, fluazifop und fluazifop-P und deren Ester, z.B. fluazifop-butyl und fluazifop-P-butyl; flucarbazone(-sodium), flucetosulfuron; fluchloralin; flufenpyr-ethyl; flumetsulam; flumeturon; flumiclorac(-pentyl), flumioxazin (S-482); flumipropyn; fluometuron, fluorochloridone, fluorodifen; fluoroglycofen(-ethyl); flupoxam (KNW-739); flupropacil (UBIC-4243); flupyrsulfuron(-methyl oder-sodium), flurenol(-butyl), fluridone; flurochloridone; fluroxypyr(-meptyl); flurprimidol, flurtamone; fluthiacet(-methyl), fluthiamide, fomesafen; foramsulfuron, fosamine; furyloxyfen; glufosinate(-ammonium); glyphosate(-isopropylammonium); halosafen; halosulfuron(-methyl) und dessen Ester (z.B. Methylester, NC-319); haloxyfop und dessen Ester; haloxyfop-P (= R-haloxyfop) und dessen Ester; hexazinone; HNPC-C9908 (Benzoic acid, 2-[[[[[4-methoxy-6-(methylthio)-2-pyrimidinyl]amino]carbonyl]amino]sulfonyl]-methylester) imazamethabenz(-methyl); imazapyr; imazaquin und Salze wie das Ammoniumsalz; imazamethapyr, imazamox, imazapic, imazethamethapyr; imazethapyr; imazosulfuron; indanofan, iodosulfuron-methyl-sodium; ioxynil; isocarbamid; isopropalin; isoproturon; isouron; isoxaben; isoxachlortole, isoxaflutole, isoxapyrifop; karbutilate; lactofen; lenacil; linuron; MCPA; MCPB; mecoprop; mefenacet; mefluidid; mesosulfuron, mesotrione, metamitron; metazachlor; methabenzthiazuron; metham; methazole; methoxyphenone; methyldymron; metabenzuron, methobenzuron; metobromuron; (alpha-)metolachlor; metosulam (XRD 511); metoxuron; metribuzin; metsulfuron-methyl; MH; molinate; monalide; monocarbamide dihydrogensulfate; monolinuron; monuron; monosulfuron; MT 128, d.h. 6-Chlor-N-(3-chlor-2-propenyl)-5-methyl-N-phenyl-3-pyridazinamin; MT 5950, d.h. N-[3-Chlor-4-(1-methylethyl)-phenyl]-2-methylpentanamid; naproanilide; napropamide; naptalam; NC 310, d.h. 4-(2,4-dichlorbenzoyl)-1-methyl-5-benzyloxypyrazol; neburon; nicosulfuron; nipyraclophen; nitralin; nitrofen; nitrofluorfen; norflurazon; orbencarb; orthosulfamuron; oryzalin; oxadiargyl (RP-020630); oxadiazon; oxasulfuron, oxaziclomefone, oxyfluorfen; paraquat; pebulate; pelargonic acid, pendimethalin; pentoxazone, perfluidone; phenisopham; phenmedipham; picloram; picolinafen, pinoxaden, piperophos; piributicarb; pirifenopbutyl; pretilachlor; primisulfuron(-methyl);, procyazine; prodiamine; profluralin; proglinazine(-ethyl); prometon; prometryn; propachlor; propanil; propaquizafop und dessen Ester; propazine; propham; propisochlor; propoxycarbazone-(sodium); propyzamide; prosulfalin; prosulfocarb; prosulfuron (CGA-152005); prynachlor; pyraflufen(-ethyl), pyrasulfotole; pyrazolinate; pyrazon; pyrazosulfuron(-ethyl); pyrazoxyfen; pyribambenz-isopropyl (ZJ0702); pyribambenz-propyl (ZJ 0273); pyribenzoxim, pyributicarb, pyridafol, pyridate; pyriftalid; pyrimidobac(-methyl), pyrimisulfan; pyrithiobac(-sodium) (KIH-2031); pyroxasulfone; pyroxofop und dessen Ester (z.B. Propargylester); pyroxsulam; quinclorac; quinmerac; quinoclamine, quinofop und dessen Esterderivate, quizalofop und quizalofop-P und deren Esterderivate z.B. quizalofop-ethyl; quizalofop-P-tefuryl und -ethyl; renriduron; rimsulfuron (DPX-E 9636); S 275, d.h. 2-[4-Chlor-2-fluor-5-(2-propynyloxy)-phenyl]-4,5,6,7-tetrahydro-2H-indazol; secbumeton; sethoxydim; siduron; simazine; simetryn; SN 106279, d.h. 2-[[7-[2-Chlor-4-(trifluor-methyl)-phenoxy]-2-naphthalenyl]-oxy]-propan-säure und -methylester; sulcotrione, sulfentrazon (FMC-97285, F-6285); sulfazuron; sulfometuron(-methyl); sulfosate (ICI-A0224); sulfosulfuron, TCA; tebutam (GCP-5544); tebuthiuron; tefuryltrione; tembotrione; tepraloxydim, terbacil; terbucarb; terbuchlor; terbumeton; terbuthylazine; terbutryn; TFH 450, d.h. N,N-Diethyl-3-[(2-ethyl-6-methylphenyl)-sulfonyl]-1H-1,2,4-triazol-1-carbox-amid; thenylchlor (NSK-850); thiafluamide, thiazafluron; thiazopyr (Mon-13200); thidiazimin (SN-24085); thiencarbazone-methyl; thifensulfuron(-methyl); thiobencarb; tiocarbazil; topramezone; tralkoxydim; tri-allate; trifloxysulfuron; triasulfuron; triaziflam, triazofenamide; tribenuron(-methyl); triclopyr; tridiphane; trietazine; trifluralin; triflusulfuron und Ester (z.B. Methylester, DPX-66037); trimeturon; tritosulfuron, tsitodef; vemolate; WL 110547, d.h. 5-Phenoxy-1-[3-(trifluormethyl)-phenyl]-1H-tetrazol; UBH-509; D-489; LS 82-556; KPP-300; NC-324; NC-330; KH-218; DPX-N8189; SC-0774; DOWCO-535; DK-8910; V-53482; PP-600; MBH-001; KIH-9201; ET-751; KIH-6127, KIH-485 und KIH-2023 Von besonderem Interesse ist die selektive Bekämpfung von Schadpflanzen in Kulturen von Nutz- und Zierpflanzen. Obgleich die erfindungsgemäßen Verbindungen (I) bereits in vielen Kulturen sehr gute bis ausreichende Selektivität aufweisen, können prinzipiell in einigen Kulturen und vor allem auch im Falle von Mischungen mit anderen Herbiziden, die weniger selektiv sind, Phytotoxizitäten an den Kulturpflanzen auftreten. Diesbezüglich sind Kombinationen erfindungsgemäßer Verbindungen der Formel (I) von besonderem Interesse, welche die Verbindungen der Formel (I) bzw. deren Kombinationen mit anderen Herbiziden oder Pestiziden und Safenem enthalten. Die Safener, welche in einem antidotisch wirksamen Gehalt eingesetzt werden, reduzieren die phytotoxischen Nebenwirkungen der eingesetzten Herbizide/Pestizide, z. B. in wirtschaftlich bedeutenden Kulturen wie Getreide (Weizen, Gerste, Roggen, Mais, Reis, Hirse), Zuckerrübe, Zuckerrohr, Raps, Baumwolle und Soja, vorzugsweise Getreide. Folgende Gruppen von Verbindungen kommen beispielsweise als Safener für die Verbindungen (I) alleinig oder aber in deren Kombinationen mit weiteren Pestiziden in Frage:
a) Verbindungen der Formeln (S-II) bis (S-IV), 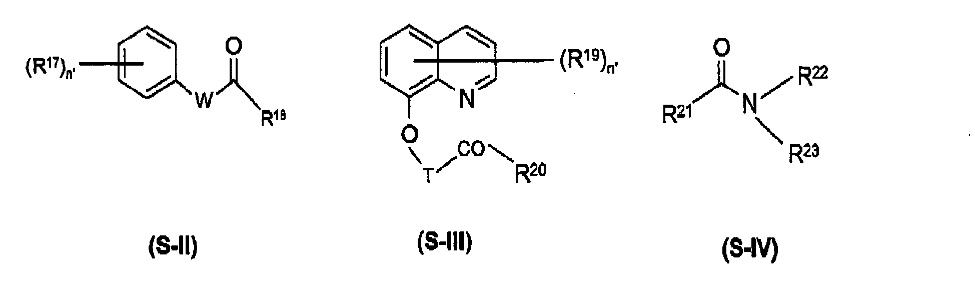 wobei die Symbole und Indizes folgende Bedeutungen haben:
   - n': ist eine natürliche Zahl von 0 bis 5, vorzugsweise 0 bis 3;
   - T: ist eine (C₁ oder C₂)-Alkandiylkette, die unsubstituiert oder mit einem oder zwei (C₁-C₄)-Alkylresten oder mit [(C₁-C₃)-Alkoxy]-carbonyl substituiert ist;
   - W: ist ein unsubstituierter oder substituierter divalenter heterocyclischer Rest aus der Gruppe der teilungesättigten oder aromatischen Fünfring-Heterocyclen mit 1 bis 3 Heteroringatomen des Typs N oder O, wobei mindestens ein N-Atom und höchstens ein O-Atom im Ring enthalten ist, vorzugsweise ein Rest aus der Gruppe (W1) bis (W4),

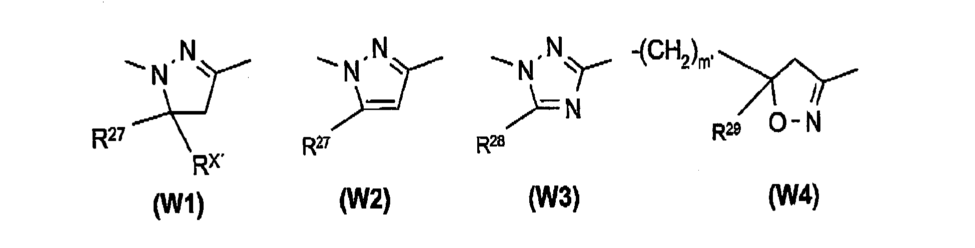

   - m': ist 0 oder 1;
   - R¹⁷, R¹⁹: sind gleich oder verschieden Halogen, (C₁-C₄)-Alkyl, (C₁-C₄)-Alkoxy, Nitro oder (C₁-C₄)-Haloalkyl;
   - R¹⁸, R²⁰: sind gleich oder verschieden OR²⁴, SR²⁴ oder NR²⁴R²⁵ oder ein gesättigter oder ungesättigter 3- bis 7-gliedriger Heterocyclus mit mindestens einem N-Atom und bis zu 3 Heteroatomen, vorzugsweise aus der Gruppe O und S, der über das N-Atom mit der Carbonylgruppe in (S-II) bzw. (S-III) verbunden ist und unsubstituiert oder durch Reste aus der Gruppe (C₁-C₄)-Alkyl, (C₁-C₄)-Alkoxy oder gegebenenfalls substituiertes Phenyl substituiert ist, vorzugsweise ein Rest der Formel OR²⁴, NHR²⁵ oder N(CH₃)₂, insbesondere der Formel OR²⁴;
   - R²⁴: ist Wasserstoff oder ein unsubstituierter oder substituierter aliphatischer Kohlenwasserstoffrest, vorzugsweise mit insgesamt 1 bis 18 C-Atomen;
   - R²⁵: ist Wasserstoff, (C₁-C₈)-Alkyl, (C₁-C₆)-Alkoxy oder substituiertes oder unsubstituiertes Phenyl;
   - R^{X'}: ist H, (C₁-C₈)-Alkyl, (C₁-C₈)-Haloalkyl, (C₁-C₄)-Alkoxy(C₁-C₈)-Alkyl, Cyano oder COOR²⁶, worin R²⁶ Wasserstoff, (C₁-C₈)-Alkyl, (C₁-C₈)-Haloalkyl, (C₁-C₄)-Alkoxy-(C₁-C₄)-alkyl, (C₁-C₆)-Hydroxyalkyl, (C₃-C₁₂)-Cycloalkyl oder Tri-(C₁-C₄)-alkyl-silyl ist;
   - R²⁷, R²⁸, R²⁹: sind gleich oder verschieden Wasserstoff, (C₁-C₈)-Alkyl, (C₁-C₈)-Haloalkyl, (C₃-C₁₂)-Cycloalkyl oder substituiertes oder unsubstituiertes Phenyl;
   - R²¹: ist (C₁-C₄)-Alkyl, (C₁-C₄)-Haloalkyl, (C₂-C₄)-Alkenyl, (C₂-C₄)-Haloalkenyl, (C₃-C₇)-Cycloalkyl, vorzugsweise Dichlormethyl;
   - R²², R²³: ist gleich oder verschieden Wasserstoff, (C₁-C₄)-Alkyl, (C₂-C₄)-Alkenyl, (C₂-C₄)-Alkinyl, (C₁-C₄)-Haloalkyl, (C₂-C₄)-Haloalkenyl, (C₁-C₄)-Alkylcarbamoyl-(C₁-C₄)-alkyl, (C₂-C₄)-Alkenylcarbamoyl-(C₁-C₄)-alkyl, (C₁-C₄)-Alkoxy-(C₁-C₄)-alkyl, Dioxolanyl-(C₁-C₄)-alkyl, Thiazolyl, Furyl, Furylalkyl, Thienyl, Piperidyl, substituiertes oder unsubstituiertes Phenyl, oder R²² und R²³ bilden zusammen einen substituierten oder unsubstituierten heterocyclischen Ring, vorzugsweise einen Oxazolidin-, Thiazolidin-, Piperidin-, Morpholin-, Hexahydropyrimidin- oder Benzoxazinring;
b) eine oder mehrere Verbindungen aus der Gruppe:
   1,8-Naphthalsäureanhydrid,
   Methyl-diphenylmethoxyacetat,
   1-(2-Chlorbenzyl)-3-(1-methyl-1-phenylethyl)hamstoff (Cumyluron),
   O,O-Diethyl S-2-ethylthioethyl phosphordithioat (Disulfoton),
   4-Chlorphenyl-methylcarbamat (Mephenate),
   O,O-Diethyl-O-phenylphosphorotioat (Dietholate),
   4-Carboxy-3,4-dihydro-2H-1-benzopyran-4-essigsäure (CL-304415, CAS-Regno: 31541-57-8),
   Cyanomethoxyimino(phenyl)acetonitril (Cyometrinil),
   1,3-Dioxolan-2-ylmethoxyimino(phenyl)acetonitril (Oxabetrinil),
   4'-Chlor-2,2,2-trifluoracetophenon-O-1,3-dioxolan-2-ylmethyloxim
   (Fluxofenim),
   4,6-Dichlor-2-phenylpyrimidin (Fenclorim),
   Benzyl-2-chlor-4-trifluormethyl-1,3-thiazol-5-carboxylat (Flurazole),
   2-Dichlormethyl-2-methyl-1,3-dioxolan (MG-191),
   N-(4-Methylphenyl)-N'-(1-methyl-1-phenylethyl)hamstoff (Dymron),
   (2,4-Dichlorphenoxy)essigsäure (2,4-D),
   (4-Chlorphenoxy)essigsäure,
   (R,S)-2-(4-Chlor-o-tolyloxy)propionsäure (Mecoprop),
   4-(2,4-Dichlorphenoxy)buttersäure (2,4-DB),
   (4-Chlor-o-tolyloxy)essigsäure (MCPA),
   4-(4-Chlor-o-tolyloxy)buttersäure,
   4-(4-Chlorphenoxy)buttersäure,
   3,6-Dichlor-2-methoxybenzoesäure (Dicamba),
   1-(Ethoxycarbonyl)ethyl 3,6-dichlor-2-methoxybenzoat (Lactidichlor)
   sowie deren Salze und Ester, vorzugsweise (C₁-C₈);
c) N-Acylsulfonamide der Formel (S-V) und ihre Salze, 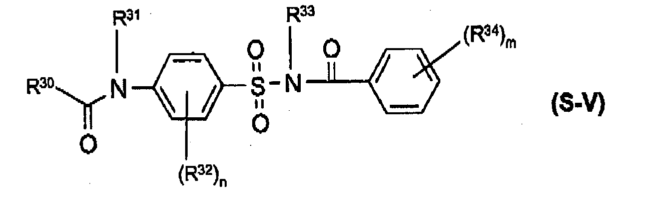 worin
   - R³⁰: Wasserstoff, einen Kohlenwasserstoffrest, einen Kohlenwasserstoffoxyrest, einen Kohlenwasserstoffthiorest oder einen Heterocyclylrest, der vorzugsweise über ein C-Atom gebunden ist, wobei jeder der letztgenannten 4 Reste unsubstituiert oder durch einen oder mehrere gleiche oder verschiedene Reste aus der Gruppe Halogen, Cyano, Nitro, Amino, Hydroxy, Carboxy, Formyl, Carbonamid, Sulfonamid und Reste der Formel -Z^{a'}-R^{a'} substituiert ist,
   wobei jeder Kohlenwasserstoffteil vorzugsweise 1 bis 20 C-Atome aufweist und ein C-haltiger Rest R³⁰ inklusive Substituenten vorzugsweise 1 bis 30 C-Atome aufweist;
   - R³¹: Wasserstoff oder (C₁-C₄)-Alkyl, vorzugsweise Wasserstoff, oder
   - R³⁰ und R³¹: zusammen mit der Gruppe der Formel -CO-N- den Rest eines 3-bis 8-gliedrigen gesättigten oder ungesättigten Rings;
   - R³²: gleich oder verschieden Halogen, Cyano, Nitro, Amino, Hydroxy, Carboxy, Formyl, CONH₂, SO₂NH₂ oder einen Rest der Formel -Z^{b'}-R^{b'};
   - R³³: Wasserstoff oder (C₁-C₄)-Alkyl, vorzugsweise H;
   - R³⁴: gleich oder verschieden Halogen, Cyano, Nitro, Amino, Hydroxy, Carboxy, CHO, CONH₂, SO₂NH₂ oder einen Rest der Formel -Z^{c'}-R^{c'};
   - R^{a'}: einen Kohlenwasserstoffrest oder einen Heterocyclylrest, wobei jeder der beiden letztgenannten Reste unsubstituiert oder durch einen oder mehrere gleiche oder verschiedene Reste aus der Gruppe Halogen, Cyano, Nitro, Amino, Hydroxy, Mono- und Di-[(C₁-C₄)-alkyl]-amino substituiert ist, oder einen Alkylrest, in dem mehrere, vorzugsweise 2 oder 3, nicht benachbarte CH₂-Gruppen jeweils durch ein Sauerstoffatom ersetzt sind;
   - R^{b'},R^{c'}: gleich oder verschieden einen Kohlenwasserstoffrest oder einen Heterocyclylrest, wobei jeder der beiden letztgenannten Reste unsubstituiert oder durch einen oder mehrere gleiche oder verschiedene Reste aus der Gruppe Halogen, Cyano, Nitro, Amino, Hydroxy, Phosphoryl, Halogen-(C₁-C₄)-alkoxy, Mono- und Di-[(C₁-C₄)-alkyl]-amino substituiert ist, oder einen Alkylrest, in dem mehrere, vorzugsweise 2 oder 3, nicht benachbarte CH₂-Gruppen jeweils durch ein Sauerstoffatom ersetzt sind;
   - Z^{a'}: eine divalente Gruppe der Formel -O-, -S-, -CO-, -CS-, -CO-O-, -CO-S-, -O-CO-, -S-CO-, -SO-, -SO₂-, -NR*-, -CO-NR*-, -NR*-CO-, -SO₂-NR*- oder -NR*-SO₂-, wobei die rechts angegebene Bindung der jeweiligen divalenten Gruppe die Bindung zum Rest R^{a'} ist und wobei die R* in den letztgenannten 5 Resten unabhängig voneinander jeweils H, (C₁-C₄)-Alkyl oder Halo-(C₁-C₄)-alkyl bedeuten;
   - Z^{b'},Z^{c'}: unabhängig voneinander eine direkte Bindung oder eine divalente Gruppe der Formel -O-, -S-, -CO-, -CS-, -CO-O-, -CO-S-, -O-CO-, -S-CO-, -SO-, -SO₂-, -NR*-, -SO₂-NR*-, -NR*-SO₂-, -CO-NR*-oder -NR*-CO-, wobei die rechts angegebene Bindung der jeweiligen divalente Gruppe die Bindung zum Rest R^{b'} bzw. R^{c'} ist und wobei die R* in den letztgenannten 5 Resten unabhängig voneinander jeweils H, (C₁-C₄)-Alkyl oder Halo-(C₁-C₄)-alkyl bedeuten;
   - n: eine ganze Zahl von 0 bis 4, vorzugsweise 0, 1 oder 2, insbesondere 0 oder 1, und
   - m: eine ganze Zahl von 0 bis 5, vorzugsweise 0, 1, 2 oder 3, insbesondere U,1 oder 2;
   bedeuten;
d) Acylsulfamoylbenzoesäureamide der allgemeinen Formel (S-VI), gegebenenfalls auch in Salzform, 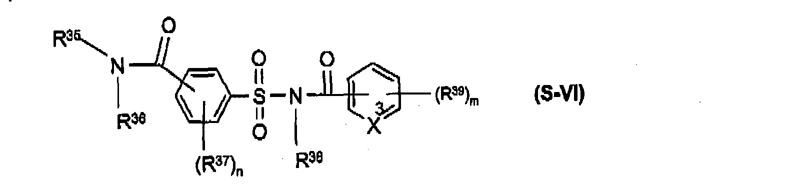 worin
   - X³: CH oder N;
   - R³⁵: Wasserstoff, Heterocyclyl oder einen Kohlenwasserstoffrest, wobei die beiden letztgenannten Reste gegebenenfalls durch einen oder mehrere, gleiche oder verschiedene Reste aus der Gruppe Halogen, Cyano, Nitro, Amino, Hydroxy, Carboxy, CHO, CONH₂, SO₂NH₂ und Z^{a'}-R^{a'} substituiert sind;
   - R³⁶: Wasserstoff, Hydroxy, (C₁-C₆)-Alkyl, (C₂-C₆)Alkenyl, (C₂-C₆)-Alkinyl, (C₁-C₆)-Alkoxy, (C₂-C₆)-Alkenyloxy, wobei die fünf letztgenannten Reste gegebenenfalls durch einen oder mehrere, gleiche oder verschiedene Reste aus der Gruppe Halogen, Hydroxy, (C₁-C₄)-Alkyl, (C₁-C₄)-Alkoxy und (C₁-C₄)-Alkylthio substituiert sind, oder
   - R³⁵ und R³⁶: zusammen mit dem sie tragenden Stickstoffatom einen 3- bis 8-gliedrigen gesättigten oder ungesättigten Ring;
   - R³⁷: Halogen, Cyano, Nitro, Amino, Hydroxy, Carboxy, CHO, CONH₂, SO₂NH₂ oder Z^{b'}-R^{b'};
   - R³⁸: Wasserstoff, (C₁-C₄)-Alkyl, (C₂-C₄)-Alkenyl oder (C₂-C₄)-Alkinyl;
   - R³⁹: Halogen, Cyano, Nitro, Amino, Hydroxy, Carboxy, Phosphoryl, CHO, CONH₂, SO₂NH₂ oder Z^{c'}-R^{c'};
   - R^{a'}: einen (C₂-C₂₀)-Alkylrest, dessen Kohlenstoffkette ein- oder mehrfach durch Sauerstoffatome unterbrochen ist, Heterocyclyl oder einen Kohlenwasserstoffrest, wobei die zwei letztgenannten Reste gegebenenfalls durch einen oder mehrere, gleiche oder verschiedene Reste aus der Gruppe Halogen, Cyano, Nitro, Amino, Hydroxy, Mono- und Di-[(C₁-C₄)-alkyl]-amino substituiert sind;
   - R^{b'},R^{c'}: gleich oder verschieden einen (C₂-C₂₀)-Alkylrest, dessen Kohlenstoffkette ein- oder mehrfach durch Sauerstoffatome unterbrochen ist, Heterocyclyl oder einen Kohlenwasserstoffrest, wobei die zwei letztgenannten Reste gegebenenfalls durch einen oder mehrere, gleiche oder verschiedene Reste aus der Gruppe Halogen, Cyano, Nitro, Amino, Hydroxy, Phosphoryl, (C₁-C₄)-Haloalkoxy, Mono- und Di-[(C₁-C₄)-alkyl]-amino substituiert sind;
   - Z^{a'}: eine divalente Einheit aus der Gruppe -O-, -S-, -CO-, -CS-, -CO-O-, -CO-S, - SO-, -SO₂-, -NR^{d'}, -CO-NR^{d'} oder -SO₂-NR^{d'};
   - Z^{b'},Z^{c'}: gleich oder verschieden eine direkte Bindung oder eine divalente Einheit aus der Gruppe-O-, -S-, -CO-, -CS-, -CO-O-, -CO-S-, -SO-, -SO₂-. -NR^{d'}, -SO₂-NR^{d'} oder-CO-NR^{d'} ;
   - R^{d'}: Wasserstoff, (C₁-C₄)-Alkyl oder (C₁-C₄)-Haloalkyl;
   - n: eine ganze Zahl von 0 bis 4, und
   - m: für den Fall, daß X für CH steht, eine ganze Zahl von 0 bis 5, und für den Fall, daß X für N steht, eine ganze Zahl von 0 bis 4 bedeuten;
e) Verbindungen vom Typ der Acylsulfamoylbenzoesäureamide, z.B. der nachfolgenden Formel (S-VII), die z.B. bekannt sind aus WO 99/16744, 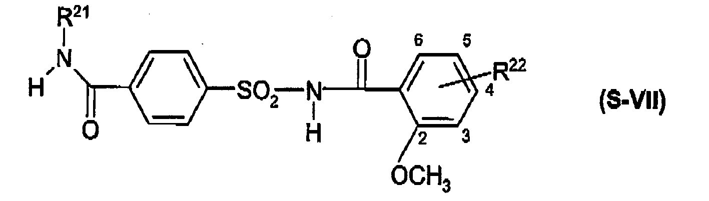 z.B. solche worin
   R²¹ = Cyclo-Propyl und R²² = H ist (S-3-1 = 4-Cyclopopylaminocarbonyl-N-(2-methoxybenzoyl)benzolsulfonamid),
   R²¹ = Cyclo-Propyl und R²² = 5-Cl ist (S-3-2),
   R²¹ = Ethyl und R²² = H ist (S-3-3),
   R²¹ = iso-Propyl und R²² = 5-Cl ist (S-3-4) und
   R²¹ = iso-Propyl und R²² = H ist (S-3-5);
f) Verbindungen vom Typ der N-Acylsulfamoylphenylharnstoffe der Formel (S-VIII), die z.B. bekannt sind aus der EP-A-365484, 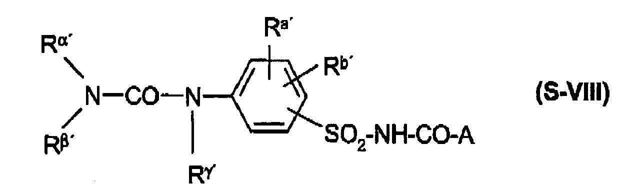 worin
   - A: für einen Rest aus der Gruppe 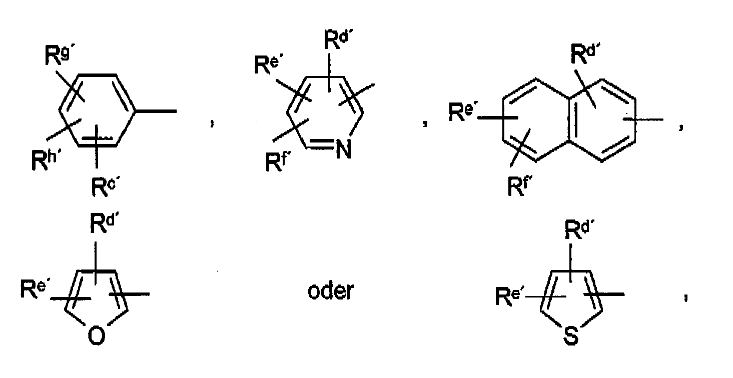
   - R^{α'} und R^{β'}: unabhängig voneinander für Wasserstoff, (C₁-C₈)-Alkyl, (C₃-C₈)-Cycloalkyl, (C₃-C₆)-Alkenyl, (C₃-C₆)-Alkinyl, 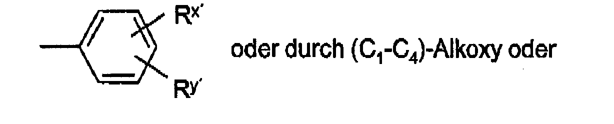 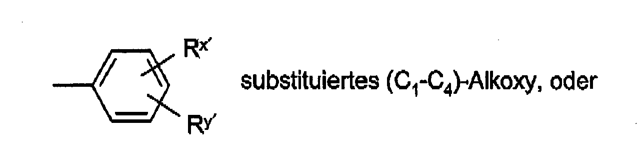
   - R^{α'} und R^{β'}: gemeinsam für eine (C₄-C₆)-Alkylenbrücke oder eine durch Sauerstoff, Schwefel, SO, SO₂, NH oder -N((C₁-C₄)-Alkyl)- unterbrochene (C₄-C₆)-Alkylenbrücke,
   - R^{γ'}: für Wasserstoff oder (C₁-C₄)-Alkyl,
   - R^{a'} und R^{b'}: unabhängig voneinander für Wasserstoff, Halogen, Cyan, Nitro, Trifluormethyl, (C₁-C₄)-Alkyl, (C₁-C₄)-Alkoxy, (C₁-C₄)-Alkylthio, (C₁-C₄)-Alkylsulfinyl, (C₁-C₄)-Alkylsulfonyl, -COORⁱ, -CONR^{k'}R^{m'}, -COR^{n'}, -SO₂NR^{k'}R^{m'} oder -OSO₂-(C₁-C₄)-Alkyl, oder R^{a'} und R^{b'} gemeinsam für eine (C₃-C₄)-Alkylenbrücke, die durch Halogen oder (C₁-C₄)-Alkyl substituiert sein kann, oder eine (C₃-C₄)-Alkenylenbrücke, die durch Halogen oder (C₁-C₄)-Alkyl substituiert sein kann, oder eine C₄-Alkadienylenbrücke, die durch Halogen oder (C₁-C₄)-Alkyl substituiert sein kann, und
   - R^{g'} und R^{h'}: unabhängig voneinander für Wasserstoff, Halogen, (C₁-C₄)-Alkyl, Trifluormethyl, Methoxy, Methylthio oder -COOR^{j'} stehen, wobei
   - R^{c'}: Wasserstoff, Halogen, (C₁-C₄)-Alkyl oder Methoxy,
   - R^{d'}: Wasserstoff, Halogen, Nitro, (C₁-C₄)-Alkyl, (C₁-C₄)-Alkoxy, (C₁-C₄)-Alkylthio, (C₁-C₄)-Alkylsulfinyl, (C₁-C₄)-Alkylsulfonyl, -COOR^{j'} oder -CONR^{k'}R^{m'},
   - R^{e'}: Wasserstoff, Halogen, (C₁-C₄)-Alkyl, -COOR^{j'}, Trifluormethyl oder Methoxy, oder R^{d'} und R^{e'} gemeinsam für eine (C₃-C₄)-Alkylenbrücke,
   - R^{f'}: Wasserstoff, Halogen oder (C₁-C₄)-Alkyl,
   - R^{X'} und R^{Y'}: unabhängig voneinander Wasserstoff, Halogen, (C₁-C₄)-Alkyl, (C₁-C₄)-Alkoxy, (C₁-C₄)-Alkylthio, -COOR^{l'}, Trifluormethyl, Nitro oder Cyan,
   - R^{j'}, R^{k'} und R^{m'}: unabhängig voneinander Wasserstoff oder (C₁-C₄)-Alkyl,
   - R^{k'} und R^{m'}: gemeinsam eine C₄-C₆-Alkylenbrücke oder eine durch Sauerstoff, NH oder -N((C₁-C₄)-Alkyl)- unterbrochene (C₄-C₆)-Alkylenbrücke, und
   - R^{n'}: (C₁-C₄)-Alkyl, Phenyl oder durch Halogen, (C₁-C₄)-Alkyl, Methoxy, Nitro oder Trifluormethyl substituiertes Phenyl bedeuten,
   bevorzugt
   1-[4-(N-2-Methoxybenzoylsulfamoyl)phenyl]-3-methylhamstoff,
   1-[4-(N-2-Methoxybenzoylsulfamoyl)phenyl]-3,3-dimethylhamstoff,
   1-[4-(N-4,5-Dimethylbenzoylsulfamoyl)phenyl]-3-methylhamstoff,
   1-[4-(N-Naphthoylsulfamoyl)phenyl]-3,3-dimethylhamstoff,
g) Verbindungen vom Typ der Acylsulfamoylbenzoesäureamide der Formel (S-IX), bekannt aus EP-A-1019368, gegebenenfalls auch in Salzform, 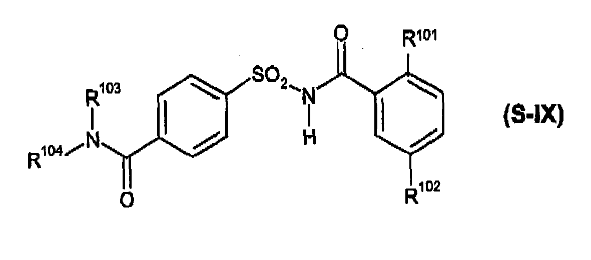 worin
   - R¹⁰¹: bedeutet Methyl, Methoxy oder Triftuormethoxy;
   - R¹⁰²: bedeutet Wasserstoff, Chlor oder Methyl;
   - R¹⁰³: bedeutet Wasserstoff, Ethyl oder Propargyl;
   - R¹⁰⁴: bedeutet Ethyl, Cyclopropyl, iso-Propyl oder Propargyl, oder
   - R¹⁰³ und R¹⁰⁴: bilden gemeinsam die Gruppe (CH₂)₄,
einschließlich der Stereoisomeren und der in der Landwirtschaft gebräuchlichen Salze.

Bevorzugt sind Herbizid-Safener-Kombinationen, enthaltend (A) eine herbizid wirksame Menge an einer oder mehrerer Verbindungen der Formel (I) oder deren Salzen, und (B) eine antidotische wirksame Menge an einem oder mehreren Safenern.

Herbizid wirksame Menge bedeutet im Sinne der Erfindung eine Menge an einem oder mehreren Herbiziden, die geeignet ist, den Pflanzenwuchs negativ zu beeinflussen. Antidotisch wirksame Menge bedeutet im Sinne der Erfindung eine Menge an einem oder mehreren Safenem, die geeignet ist, die phytotoxische Wirkung von Pflanzenschutzmittelwirkstoffen (z.B. von Herbiziden) an Kulturpflanzen zu reduzieren.
Die Verbindungen der Formel (S-II) sind z.B. aus EP-A-0 333131 (ZA-89/1960), EP-A-0 269 806 (US-A-4,891,057), EP-A-0 346 620 (AU-A-89134951), EP-A-0 174 562, EP-A-0 346 620 (WO-A-91/08 202), WO-A-91/07 874 oder WO-A 95/07 897 (ZA 94/7120) und der dort zitierten Literatur bekannt oder können nach oder analog den dort beschriebenen Verfahren hergestellt werden. Die Verbindungen der Formel (S-III) sind aus EP-A-0 086 750, EP-A-0 94349 (US-A-4,902,340), EP-A-0 191736 (US-A-4,881,966) und EP-A-0 492 366 und dort zitierter Literatur bekannt oder können nach oder analog den dort beschriebenen Verfahren hergestellt werden.

Einige Verbindungen sind ferner in EP-A-0 582 198 und WO 2002/34048 beschrieben.
Die Verbindungen der Formel (S-IV) sind aus zahlreichen Patentanmeldungen bekannt, beispielsweise US-A-4,021,224 und US-A-4,021,229.
Verbindungen der Gruppe B (b) sind weiterhin aus CN-A- 87/102 789, EP-A-365484 sowie aus "The Pesticide Manual", The British Crop Protection Council and the Royal Society of Chemistry, 11th edition, Famham 1997, bekannt.
Die Verbindungen der Gruppe B (c) sind in der WO-A-97/45016, die der Gruppe B (d) in der WO-A-99/16744, die der Gruppe B (e) in der EP-A-365484 und die der Gruppe B (g) in EP-A-1019368 beschrieben.
Die zitierten Schriften enthalten ausführliche Angaben zu Herstellungsverfahren und Ausgangsmaterialien und nennen bevorzugte Verbindungen. Auf diese Schriften wird ausdrücklich Bezug genommen, sie gelten durch Zitat als Bestandteil dieser Beschreibung.
Bevorzugt sind Herbizid-Safener Kombinationen, enthaltend Safener der Formel (S-II) und/oder (S-III) bei denen die Symbole und Indizes folgende Bedeutungen haben:
- R²⁴: ist Wasserstoff, (C₁-C₁₈)-Alkyl, (C₃-C₁₂)-Cycloalkyl, (C₂-C₈)-Alkenyl und (C₂-C₁₈)-Alkinyl, wobei die C-haltigen Gruppen durch einen oder mehrere, vorzugsweise bis zu drei, Reste R⁵⁰ substituiert sein können;
- R⁵⁰: ist gleich oder verschieden Halogen, Hydroxy, (C₁-C₈)-Alkoxy, (C₁-C₈)-Alkylthio, (C₂-C₈)-Alkenylthio, (C₂-C₈)-Alkinylthio, (C₂-C₈)-Alkenyloxy, (C₂-C₈)-Alkinyloxy, (C₃-C₇)-Cycloalkyl, (C₃-C₇)-Cycloalkoxy, Cyano, Mono- und Di-((C₁-C₄)-alkyl)-amino, Carboxy, (C₁-C₈)-Alkoxycarbonyl, (C₂-C₈)-Alkenyloxycarbonyl, (C₁-C₈)Alkylthiocarbonyl, (C₂-C₈)-Alkinyloxycarbonyl, (C₁-C₈)-Alkylcarbonyl, (C₂-C₈)-Alkenylcarbonyl, (C₂-C₈)-Alkinylcarbonyl, 1-(Hydroxyimino)-(C₁-C₆)-alkyl, 1-[(C₁-C₄)-Alkylimino]-(C₁-C₄)-alkyl, 1-[(C₁-C₄)-Alkoxyimino]-(C₁-C₆)-alkyl, (C₁-C₈)-Alkylcarbonylamino, (C₂-C₈)-Alkenylcarbonylamino, (C₂-C₈)-Alkinylcarbonylamino, Aminocarbonyl, (C₁-C₈)-Alkylaminocarbonyl, Di-((C₁-C₆)-alkyl)-aminocarbonyl, (C₂-C₆)-Alkenylaminocarbonyl, (C₂-C₆)-Alkinylaminocarbonyl, (C₁-C₈)-Alkoxycarbonylamino, (C₁-C₈)-Alkylaminocarbonylamino, (C₁-C₆)-Alkylcarbonyloxy, das unsubstituiert oder durch R⁵¹ substituiert ist, (C₂-C₆)-Alkenylcarbonyloxy, (C₂-C₆)-Alkinylcarbonyloxy, (C₁-C₈)-Alkylsulfonyl, Phenyl, Phenyl-(C₁-C₆)-alkoxy, Phenyl-(C₁-C₆)-alkoxycarbonyl, Phenoxy, Phenoxy-(C₁-C₆)-alkoxy, Phenoxy-(C₁-C₆)-alkoxycarbonyl, Phenylcarbonyloxy, Phenylcarbonylamino, Phenyl-(C₁-C₈)-alkylcarbonylamino, wobei die letztgenannten 9 Reste im Phenylring unsubstituiert oder ein- oder mehrfach, vorzugsweise bis zu dreifach durch Reste R⁵² substituiert sind; SiR'₃, -O-SiR'₃, R'₃Si-(C₁-C₈)-alkoxy, -CO-O-NR'₂, -O-N=CR'₂, -N=CR'₂, -O-NR'2, -NR'₂, CH(OR')₂, -O-(CH₂)ₘ -CH(OR')₂, -CR"'(OR')₂, -O-(CH₂)ₘCR"'(OR")₂ oder durch R"O-CHR"'CHCOR"-(C₁-C₆)-alkoxy,
- R⁵¹: ist gleich oder verschieden Halogen, Nitro, (C₁-C₄)-Alkoxy und unsubstituiertes oder mit einem oder mehreren, vorzugsweise bis zu drei Resten R⁵² substituiertes Phenyl;
- R⁵²: ist gleich oder verschieden Halogen, (C₁-C₄)-Alkyl, (C₁-C₄)-Alkoxy, (C₁-C₄)-Haloalkyl, (C₁-C₄)-Haloalkoxy oder Nitro;
- R': ist gleich oder verschieden Wasserstoff, (C₁-C₄)-Alkyl, unsubstituiertes oder durch einen oder mehrere, vorzugsweise bis zu drei, Reste R⁵² substituiertes Phenyl oder zwei Reste R' bilden zusammen eine (C₂-C₆)-Alkandiylkette;
- R": ist gleich oder verschieden (C₁-C₄)-Alkyl oder zwei Reste R" bilden zusammen eine (C₂-C₆)-Alkandiylkette;
- R"': ist Wasserstoff oder (C₁-C₄)-Alkyl;
- m: ist 0, 1, 2, 3, 4, 5 oder 6.

Besonders bevorzugt sind erfindungsgemäße Herbizid-Safener-Kombinationen, enthaltend Safener der Formel (S-II) und/oder (S-III), bei denen die Symbole und Indizes folgende Bedeutungen haben:
- R²⁴: ist Wasserstoff, (C₁-C₈)-Alkyl oder (C₃-C-₇)-Cycloalkyl, wobei die vorstehenden C-haltigen Reste unsubstituiert sind oder ein- oder mehrfach durch Halogen oder ein- oder zweifach, vorzugsweise einfach, durch Reste R⁵⁰ substituiert sind,
- R⁵⁰: ist gleich oder verschieden Hydroxy, (C₁-C₄)-Alkoxy, Carboxy, (C₁-C₄)-Alkoxycarbonyl, (C₂-C₆)-Alkenyloxycarbonyl, (C₂-C₆)-Alkinyloxycarbonyl, 1-(Hydroxyimino)-(C₁-C₄)-alkyl, 1-[(C₁-C₄)Alkylimino]-(C₁-C₄)-alkyl und 1-[(C₁-C₄)-Alkoxyimino]-(C₁-C₄)-alkyl; -SiR'₃, -O-N=CR'₂, -N=CR'₂, -NR'₂, und -O-NR'₂, worin R' gleich oder verschieden Wasserstoff, (C₁-C₄)-Alkyl oder paarweise eine (C₄-C₅)-Alkandiylkette bedeutet,
- R²⁷, R²⁸, R²⁹: sind gleich oder verschieden Wasserstoff, (C₁-C₈)-Alkyl, (C₁-C₆)-Haloalkyl, (C₃-C₇)-Cycloalkyl oder Phenyl, das unsubstituiert oder durch einen oder mehrere der Reste aus der Gruppe Halogen, Cyano, Nitro, Amino, Mono- und Di-[(C₁-C₄)-alkyl]-amino, (C₁-C₄)-Alkyl, (C₁-C₄)-Haloalkyl, (C₁-C₄)-Alkoxy, (C₁-C₄)-Haloalkoxy, (C₁-C₄)-Alkylthio und (C₁-C₄)-Alkylsulfonyl substituiert ist;
- R^{X'}: ist Wasserstoff oder COOR²⁴, worin R²⁶ Wasserstoff, (C₁-C₈)-Alkyl, (C₁-C₈)-Haloalkyl, (C₁-C₄)-Alkoxy-(C₁-C₄)-alkyl, (C₁-C₆)-Hydroxyalkyl, (C₃-C₇)-Cycloalkyl oder Tri-(C₁-C₄)-alkylsilyl bedeutet,
- R¹⁷, R¹⁹: sind gleich oder verschieden Halogen, Methyl, Ethyl, Methoxy, Ethoxy, (C₁ oder C₂)-Haloalkyl, vorzugsweise Wasserstoff, Halogen oder (C₁ oder C₂)-Haloalkyl.

Ganz besonders bevorzugt sind Safener in welchen die Symbole und Indizes in Formel (S-II) folgende Bedeutungen haben:
- R¹⁷: ist Halogen, Nitro oder (C₁-C₄)-Haloalkyl;
- n': ist 0, 1, 2 oder 3;
- R¹⁸: ist ein Rest der Formel OR²⁴,
- R²⁴: ist Wasserstoff, (C₁-C₈)-Alkyl oder (C₃-C₇)-Cycloalkyl, wobei die vorstehenden C-haitigen Reste unsubstituiert sind oder ein- oder mehrfach, vorzugsweise bis zu dreifach, durch gleiche oder verschiedene Halogen-Reste oder bis zu zweifach, vorzugsweise einfach, durch gleiche oder verschiedene Reste aus der Gruppe Hydroxy, (C₁-C₄)-Alkoxy, (C₁-C₄)-Alkoxycarbonyl, (C₂-C₆)-Alkenyloxycarbonyl, (C₂-C₆)-Alkinyloxycarbonyl, 1-(Hydroxyimino)-(C₁-C₄)-alkyl, 1-[(C₁-C₄)-Alkylimino]-(C₁-C₄)-alkyl, 1-[(C₁-C₄)-Alkoxyimino]-(C₁-C₄)alkyl und Reste der Formeln -SiR'₃, -O-N=R'₂, -N=CR'₂, -NR'₂ und -O-NR'₂ substituiert sind, wobei die Reste R' in den genannten Formeln gleich oder verschieden Wasserstoff, (C₁-C₄)-Alkyl oder paarweise (C₄ oder C₆)-Alkandiyl bedeuten;
- R²⁷, R²⁸, R²⁹: sind gleich oder verschieden Wasserstoff, (C₁-C₈)-Alkyl, (C₁-C₆)-Haloalkyl, (C₃-C₇)-Cycloalkyl oder Phenyl, das unsubstituiert oder durch einen oder mehrere der Reste aus der Gruppe Halogen, (C₁-C₄)-Alkyl, (C₁-C₄)-Alkoxy, Nitro, (C₁-C₄)-Haloalkyl und (C₁-C₄)-Haloalkoxy substituiert ist, und
- R^{X'}: ist Wasserstoff oder COOR²⁶, worin R²⁶ Wasserstoff, (C₁-C₈)-Alkyl, (C₁-C₈)-Haloalkyl, (C₁-C₄)-Alkoxy-(C₁-C₄)-alkyl, (C₁-C₆)-Hydroxyalkyl, (C₃-C₇)-Cycloalkyl oder Tri-(C₁-C₄)-alkylsilyl ist.

Ganz besonders bevorzugt sind auch Safener der Formel (S-III), bei denen die Symbole und Indizes folgende Bedeutungen haben:
- R¹⁹: ist Halogen oder (C₁-C₄)-Haloalkyl;
- n': ist 0, 1, 2 oder 3, wobei (R¹⁹)_{n'} vorzugsweise 5-Cl ist;
- R²⁰: ist ein Rest der Formel OR²⁴;
- T: ist CH₂ oder CH(COO-((C₁-C₃)-Alkyl)) und
- R²⁴: ist Wasserstoff, (C₁-C₈)-Alkyl, (C₁-C₈)-Haloalkyl oder (C₁-C₄)-Alkoxy-(C₁-C₄)-alkyl, vorzugsweise Wasserstoff oder (C₁-C₈)-Alkyl.

Insbesondere bevorzugt sind dabei Safener der Formel (II) bei denen die Symbole und Indizes folgende Bedeutungen haben:
- W: ist (W1);
- R¹⁷: ist Halogen oder (C₁-C₂)-Haloalkyl;
- n': ist 0,1, 2 oder 3, wobei (R¹⁷)_{n'} vorzugsweise 2,4-Cl₂ ist;
- R¹⁸: ist ein Rest der Formel OR²⁴;
- R²⁴: ist Wasserstoff, (C₁-C₈)-Alkyl, (C₁-C₄)-Haloalkyl, (C₁-C₄)-Hydroxyalkyl, (C₃-C₇)-Cycloalkyl, (C₁-C₄)-Alkoxy-(C₁-C₄)-alkyl oder Tri-(C₁-C₂)-alkylsilyl, vorzugsweise (C₁-C₄)-Alkyl;
- R²⁷: ist Wasserstoff, (C₁-C₈)-Alkyl, (C₁-C₄)-Haloalkyl oder (C₃-C₇)-Cycloalkyl, vorzugsweise Wasserstoff oder (C₁-C₄)-Alkyl, und
- R^{X'}: ist COOR²⁶, worin R²⁶ Wasserstoff, (C₁-G₈)-Alkyl, (C₁-C₄)-Haloalkyl, (C₁-C₄)-Hydroxyalkyl, (C₃-C₇)-Cycloalkyl, (C₁-C₄)-Alkoxy-(C₁-C₄)-alkyl oder Tri-(C₁-C₂)-alkylsilyl, vorzugsweise Wasserstoff oder (C₁-C₄)-Alkyl ist.

Insbesondere bevorzugt sind auch herbizide Mittel, enthaltend einen Safener der Formel (S-II), worin die Symbole und Indizes folgende Bedeutungen haben:
- W: ist (W2);
- R¹⁷: ist Halogen oder (C₁-C₂)-Haloalkyl;
- n': ist 0, 1, 2 oder 3, wobei (R¹⁷)_{n'} vorzugsweise 2,4-Cl₂ ist;
- R¹⁸: ist ein Rest der Formel OR²⁴;
- R²⁴: ist Wasserstoff, (C₁-C₈)-Alkyl, (C₁-C₄)-Haloalkyl, (C₁-C₄)-Hydroxyalkyl, (C₃-C₇)-Cycloalkyl, ((C₁-C₄)-Alkoxy)-(C₁-C₄)-alkyl oder Tri-(C₁-C₂)-alkyl-silyl, vorzugsweise (C₁-C₄)-Alkyl, und
- R²⁷: ist Wasserstoff, (C₁-C₈)-Alkyl, (C₁-C₄)-Haloalkyl, (C₃-C₇)-Cycloalkyl oder unsubstituiertes oder substituiertes Phenyl, vorzugsweise Wasserstoff, (C₁-C₄)-Alkyl oder Phenyl, das unsubstituiert oder durch einen oder mehrere Reste aus der Gruppe Halogen, (C₁-C₄)-Alkyl, (C₁-C₄)-Haloalkyl, Nitro, Cyano oder (C₁-C₄)-Alkoxy substituiert ist.

Insbesondere bevorzugt sind auch Safener der Formel (S-II), worin die Symbole und Indizes folgende Bedeutungen haben:
- W: ist (W3);
- R¹⁷: ist Halogen oder (C₁-C₂)-Haloalkyl;
- n': ist 0, 1, 2 oder 3, wobei (R¹⁷)_{n'} vorzugsweise 2,4-Cl₂ ist;
- R¹⁸: ist ein Rest der Formel OR²⁴;
- R²⁴: ist Wasserstoff, (C₁-C₈)-Alkyl, (C₁-C₄)-Haloalkyl, (C₁-C₄)-Hydroxyalkyl, (C₃-C₇)-Cycloalkyl, (C₁-C₄)-Alkoxy-(C₁-C₄)-alkyl oder Tri-(C₁-C₂)-alkylsilyl, vorzugsweise (C₁-C₄)-Alkyl, und
- R²⁸: ist (C₁-C₈)-Alkyl oder (C₁-C₄)-Haloalkyl, vorzugsweise C₁-Haloalkyl.

Insbesondere bevorzugt sind auch Safener der Formel (S-II), worin die Symbole und Indizes folgende Bedeutung haben:
- W: ist (W4);
- R¹⁷: ist Halogen, Nitro, (C₁-C₄)-Alkyl, (C₁-C₂)-Haloalkyl, vorzugsweise CF₃, oder (C₁-C₄)-Alkoxy;
- n': ist 0, 1, 2 oder 3;
- m': ist 0 oder 1;
- R¹⁸: ist ein Rest der Formel OR²⁴;
- R²⁴: ist Wasserstoff, (C₁-C₄)-Alkyl, Carboxy-(C₁-C₄)-alkyl, (C₁-C₄)-Alkoxycarbonyl-(C₁-C₄)-alkyl, vorzugsweise (C₁-C₄)-Alkoxy-CO-CH₂-, (C₁-C₄)-Alkoxy-CO-C(CH₃)H-, HO-CO-CH₂- oder HO-CO-C(CH₃)H-, und
- R²⁹: ist Wasserstoff, (C₁-C₄)-Alkyl, (C₁-C₄)-Haloalkyl, (C₃-C₇)-Cycloalkyl oder Phenyl, das unsubstituiert oder durch einen oder mehrere der Reste aus der Gruppe Halogen, (C₁-C₄)-Alkyl, (C₁-C₄)-Haloalkyl, Nitro, Cyano und (C₁-C₄)-Alkoxy substituiert ist.

Folgende Gruppen von Verbindungen sind insbesondere als Safener für die herbiziden Wirkstoffe der Formel (I) geeignet:
b) Verbindungen vom Typ der Dichlorphenylpyrazolin-3-carbonsäure (d.h. der Formel (S-II), worin W = (W1) und (R¹⁷)_{n'} = 2,4-Cl₂), vorzugsweise Verbindungen wie 1-(2,4-Dichlorphenyl)-5-(ethoxycarbonyl)-5-methyl-2-pyrazolin-3-carbonsäureethylester (S-II-1, Mefenpyr-diethyl), Mefenpyrdimethyl und Mefenpyr (S-II-0), und verwandte Verbindungen, wie sie in der WO-A 91/07874 beschrieben sind;
c) Derivate der Dichlorphenylpyrazolcarbonsäure (d.h. der Formel (S-II), worin W = (W2) und (R¹⁷)_{n'} = 2,4-Cl₂ ist), vorzugsweise Verbindungen wie 1-(2,4-Dichlorphenyl)-5-methyl-pyrazol-3-carbonsäureethylester (S-II-2), 1-(2,4-Dichlorphenyl)-5-isopropyl-pyrazol-3-carbonsäureethylester (S-II-3), 1-(2,4-Dichlorphenyl)-5-(1,1-dimethyl-ethyl)pyrazol-3-carbonsäureethylester (S-II-4), 1-(2,4-Dichlorphenyl)-5-phenyl-pyrazol-3-carbonsäureethylester (S-II-5) und verwandte Verbindungen, wie sie in EP-A-0 333131 und EP-A-0 269 806 beschrieben sind;
d) Verbindungen vom Typ der Triazolcarbonsäuren (d.h. der Formel (S-II), worin W = (W3) und (R¹⁷)_{n'} = 2,4-Cl₂ ist), vorzugsweise Verbindungen wie Fenchlorazol-ethyl, d.h. 1-(2,4-Dichlorphenyl)-5-trichlormethyl-(1H)-1,2,4-triazol-3-carbonsäureethylester (S-II-6), und verwandte Verbindungen (siehe EP-A-0 174 562 und EP-A-0 346 620);
e) Verbindungen vom Typ der 5-Benzyl- oder 5-Phenyl-2-isoxazolin-3-carbonsäure oder der 5,5-Diphenyl-2-isoxazolin-3-carbonsäure wie Isoxadifen (S-II-12), (worin W = (W4) ist), vorzugsweise Verbindungen wie 5-(2,4-Dichlorbenzyl)-2-isoxazolin-3-carbonsäureethylester (S-II-7) oder 5-Phenyl-2-isoxazolin-3-carbonsäureethylester (S-II-8) und verwandte Verbindungen, wie sie in WO-A- 91/08202 beschrieben sind, oder der 5,5-Diphenyl-2-isoxazolin-carbonsäureethylester (S-II-9, Isoxadifen-ethyl) oder -n-propylester (S-II-10) oder der 5-(4-Fluorphenyl)-5-phenyl-2-isoxazolin-3-carbonsäureethylester (S-II-11), wie sie in der WO-A-95/07897 beschrieben sind.
e) Verbindungen vom Typ der 8-Chinolinoxyessigsäure, z.B. solche der Formel (S-III), worin (R¹⁹)_{n'} = 5-Cl, R²⁰ = OR²⁴ und T = CH₂ ist, vorzugsweise die Verbindungen
   (5-Chlor-8-chinolinoxy)essigsäure-(1-methylhexyl)-ester (S-III-1, Cloquintocet-mexyl),
   (5-Chlor-8-chinolinoxy)essigsäure-(1,3-dimethyl-but-1-yl)-ester (S-III-2),
   (5-Chlor-8-chinolinoxy)essigsäure-4-allyl-oxy-butylester (S-III-3),
   (5-Chlor-8-chinolinoxy)essigsäure-1-allyloxy-prop-2-ylester (S-III-4),
   (5-Chlor-8-chinolinoxy)essigsäureethylester (S-III-5),
   (5-Chlor-8-chinolinoxy)essigsäuremethylester (S-III-6),
   (5-Chlor-8-chinolinoxy)essigsäureallylester (S-III-7),
   (5-Chlor-8-chinolinoxy)essigsäure-2-(2-propyliden-iminoxy)-1-ethylester (S-III-8),
   (5-Chlor-8-chinolinoxy)essigsäure-2-oxo-prop-1-ylester (S-III-9), (5-Chlor-8-chinolinoxy)essigsäure (S-III-10) und dessen Salze wie sie z.B. in der WO-A-2002/34048 beschrieben sind,
   und verwandte Verbindungen, wie sie in EP-A-0 860 750, EP-A-0 094 349
   und EP-A-0 191 736 oder EP-A-0 492 366 beschrieben sind.
g) Verbindungen vom Typ der (5-Chlor-8-chinolinoxy)-malonsäure, d.h. der Formel (S-III), worin (R¹⁹)_{n'} = 5-Cl, R²⁰= OR²⁴, T = -CH(COO-Alkyl)- ist, vorzugsweise die Verbindungen (5-Chlor-8-chinolinoxy)malonsäurediethylester (S-III-11), (5-Chlor-8-chinolinoxy)-malonsäurediallylester, (5-Chlor-8-chinolinoxy)-malonsäure-methyl-ethylester und verwandte Verbindungen, wie sie in EP-A-0 582 198 beschrieben sind.
h) Verbindungen vom Typ der Dichloracetamide, d.h. der Formel (S-IV), vorzugsweise:
   N,N-Diallyl-2,2-dichloracetamid (Dichlormid (S-IV-1), aus US-A 4,137,070),
   4-Dichloracetyl-3,4-dihydro-3-methyl-2H-1,4-benzoxazin (IV-2, Benoxacor, aus EP 0 149 974),
   N1,N2-Diallyl-N2-dichloracetylglycinamid (DKA-24 (IV-3), aus HU 2143821),
   4-Dichloracetyl-1-oxa-4-aza-spiro[4,5]decan (AD-67),
   2,2-Dichlor-N-(1,3-dioxolan-2-ylmethyl)-N-(2-propenyl)acetamid (PPG-1292),
   3-Dichloracetyl-2,2,5-trimethyloxazolidin (R-29148, S-IV-4),
   3-Dichloracetyl-2,2-dimethyl-5-phenyloxazolidin,
   3-Dichloracetyl-2,2-dimethyl-5-(2-thienyl)oxazolidin,
   3-Dichloracetyl-5-(2-furanyl)2,2-dimethyloxazolidin (Furilazole (S-IV-5), MON 13900),
   1-Dichloracetyl-hexahydro-3,3,8a-trimethylpyrrolo[1,2-a]pyrimidin-6(2H)-on (Dicyclonon, BAS 145138),
i) Verbindungen der Gruppe B(b), vorzugsweise
   1,8-Naphthalsäureanhydrid (S-b-1).
   Methyl-diphenylmethoxyacetat (S-b-2),
   Cyanomethoxyimino(phenyl)acetonitril (Cyometrinli) (S-b-3),
   1-(2-Chlorbenzyl)-3-(1-methyl-1-phenylethyl)harnstoff (Cumyluron) (S-b-4),
   O,O-Diethyl S-2-ethylthioethyl phosphordithioat (Disulfoton) (S-b-5),
   4-Chlorphenyl-methylcarbamat (Mephenate) (S-b-6),
   O,O-Diethyl-O-phenylphosphorotioat (Dietholate) (S-b-7),
   4-Carboxy-3,4-dihydro-2H-1-benzopyran-4-essigsäure (CL-304415, CAS-Regno: 31541-57-8) (S-b-8),
   1,3-Dioxolan-2-ylmethoxyimino(phenyl)acetonitril (Oxabetrinil) (S-b-9),
   4'-Chlor-2,2,2-trifluoracetophenon-O-1,3-dioxolan-2-ylmethyloxim (Fluxofenim) (S-b-10),
   4,6-Dichlor-2-phenylpyrimidin (Fenclorim) (S-b-11),
   Benzyl-2-chlor-4-trifluormethyl-1,3-thiazol-5-carboxylat (Flurazole) (S-b-12),
   2-Dichlormethyl-2-methyl-1,3-dioxolan (MG-191) (S-b-13).
   N-(4-Methylphenyl)-N'-(1-methyl-1-phenylethyl)hamstoff (Dymron) (S-b-14), (2,4-Dichlorphenoxy)essigsäure (2,4-D),
   (4-Chlorphenoxy)essigsäure,(R,S)-2-(4-Chlor-o-tolyloxy)propionsäure (Mecoprop),
   4-(2,4-Dichlorphenoxy)buttersäure (2,4-DB),
   (4-Chlor-o-tolyloxy)essigsäure (MCPA),
   4-(4-Chlor-o-tolyloxy)buttersäure,
   4-(4-Chlorphenoxy)buttersäure,
   3,6-Dichlor-2-methoxybenzoesäure (Dicamba),
   1-(Ethoxycarbonyl)ethyl 3,6-dichlor-2-methoxybenzoat (Lactidichlor)
sowie deren Salze und Ester, vorzugsweise (C₁-C₈).

Bevorzugt sind als Safener weiterhin Verbindungen der Formel (S-V) oder deren Salze, worin
- R³⁰: Wasserstoff, (C₁-C₆)-Alkyl, (C₃-C₆)-Cycloalkyl, Furanyl oder Thienyl, wobei jeder der letztgenannten 4 Reste unsubstituiert oder durch einen oder mehrere Substituenten aus der Gruppe Halogen, (C₁-C₄)-Alkoxy, Halogen-(C₁-C₆)-alkoxy und (C₁-C₄)-Alkylthio und im Falle cyclischer Reste auch (C₁-C₄)-Alkyl und (C₁-C₄)-Haloalkyl substituiert ist,

- R³¹: Wasserstoff,
- R³²: Halogen, Halogen-(C₁-C₄)-alkyl, Halogen-(C₁-C₄)-alkoxy, (C₁-C₄)-Alkyl, (C₁-C₄)-Alkoxy, (C₁-C₄)-Alkylsulfonyl, (C₁-C₄)-Alkoxycarbonyl oder (C₁-C₄)-Alkylcarbonyl,
vorzugsweise Halogen, (C₁-C₄)-Halogenalkyl, wie Trifluormethyl, (C₁-C₄)-Alkoxy, Halogen-(C₁-C₄)-alkoxy, (C₁-C₄)-Alkoxycarbonyl oder (C₁-C₄)-Alkylsulfonyl,
- R³³: Wasserstoff,
- R³⁴: Halogen, (C₁-C₄)-Alkyl, Halogen-(C₁-C₄)-alkyl, Halogen-(C₁-C₄)-alkoxy, (C₃-C₆)-Cycloalkyl, Phenyl, (C₁-C₄)-Alkoxy, Cyano, (C₁-C₄)-Alkylthio, (C₁-C₄)-Alkylsulfinyl, (C₁-C₄)-Alkylsulfonyl, (C₁-C₄)-Alkoxycarbonyl oder (C₁-C₄)-Alkylcarbonyl,
vorzugsweise Halogen, (C₁-C₄)-Alkyl, (C₁-C₄)-Halogenalkyl, wie Trifluormethyl, Halogen-(C₁-C₄)-alkoxy, (C₁-C₄)-Alkoxy oder (C₁-C₄)-Alkylthio,
- n: 0, 1 oder 2 und
- m: 1 oder 2 bedeuten.

Besonders bevorzugt sind Verbindungen der Formel (S-V), worin
R³⁰ = H₃C-O-CH₂-, R³¹ = R³³ = H, R³⁴ = 2-OMe ist (S-V-1),
R³⁰ = H₃C-O-CH₂-, R³¹ = R³³ = H, R³⁴ = 2-OMe-5-Cl ist (S-V-2),
R³⁰ = Cyclopropyl, R³¹ = R³³ = H, R³⁴ = 2-OMe ist (S-V-3),
R³⁰ = Cyclopropyl, R³¹ = R³³ = H, R³⁴ = 2-OMe-5-Cl ist (S-V-4),
R³⁰ = Cyclopropyl, R³¹ = R³³ = H, R³⁴ = 2-Me ist (S-V-5),
R³⁰ = tert. Butyl, R³¹ = R³³ = H, R³⁴ = 2-OMe ist (S-V-6).

Weiterhin bevorzugt sind Safener der Formel (S-V1), in der
- X³: CH;
- R³⁵: Wasserstoff, (C₁-C₆)-Alkyl, (C₃-C₆)-Cycloalkyl, (C₂-C₆)-Alkenyl, (C₅-C₆)-Cycloalkenyl, Phenyl oder 3- bis 6-gliedriges Heterocyclyl mit bis zu drei Heteroatomen aus der Gruppe Stickstoff, Sauerstoff und Schwefel, wobei die sechs letztgenannten Reste gegebenenfalls durch einen oder mehrere, gleiche oder verschiedene Substituenten aus der Gruppe Halogen, (C₁-C₆)-Alkoxy, (C₁-C₆)-Haloalkoxy, (C₁-C₂)-Alkylsulfinyl, (C₁-C₂)-Alkylsulfonyl, (C₃-C₆)-Cycloalkyl, (C₁-C₄)-Alkoxycarbonyl, (C₁-C₄)-Alkylcarbonyl und Phenyl und im Falle cyclischer Reste auch (C₁-C₄)-Alkyl und (C₁-C₄)-Haloalkyl substituiert sind;
- R³⁶: Wasserstoff, (C₁-C₆)-Alkyl, (C₂-C₆)-Alkenyl, (C₂-C₆)-Alkinyl, wobei die drei letztgenannten Reste gegebenenfalls durch einen oder mehrere, gleiche oder verschiedene Substituenten aus der Gruppe Halogen, Hydroxy, (C₁-C₄)-Alkyl, (C₁-C₄)-Alkoxy und (C₁-C₄)-Alkylthio substituiert sind;
- R³⁷: Halogen, (C₁-C₄)-Haloalkyl, (C₁-C₄)-Haloalkoxy, Nitro, (C₁-C₄)-Alkyl, (C₁-C₄)-Alkoxy, (C₁-C₄)-Alkylsulfonyl, (C₁-C₄)-Alkoxycarbonyl oder (C₁-C₄)-Alkylcarbonyl;
- R³⁸: Wasserstoff;
- R³⁸: Halogen, Nitro, (C₁-C₄)-Alkyl, (C₁-C₄)-Haloalkyl, (C₁-C₄)-Haloalkoxy, (C₃-C₆)-Cycloalkyl, Phenyl, (C₁-C₄)-Alkoxy, Cyano, (C₁-C₄)-Alkylthio, (C₁-C₄)-Alkylsulfinyl, (C₁-C₄)-Alkylsulfonyl, (C₁-C₄)-Alkoxycarbonyl oder (C₁-C₄)-Alkylcarbonyl;
- n: 0, 1 oder 2 und
- m: 1 oder 2
bedeuten.

Bevorzugte Safener der Formel (S-VII) sind (S-3-1), (S-3-2), (S-3-3), (S-3-4) und (S-3-5).
Bevorzugte Safener der Formel (VIII) sind
1-[4-(N-2-Methoxybenzoylsulfamoyl)phenyl]-3-methylhamstoff (S-VIII-1),
1-[4-(N-2-Methoxybenzoylsulfamoyl)phenyl]-3,3-dimethylhamstoff (S-VIII-2),
1-[4-(N-4,5-Dimethylbenzoylsulfamoyl)phenyl]-3-methylhamstoff (S-VIII-3) und
1-[4-(N-Naphthoylsulfamoyl)phenyl]-3,3-dimethylhamstoff (S-VIII-4).

Bevorzugte Safener der Formel S-IX sind sind Verbindungen der Formeln S-IX-A1 bis S-IX-A4, 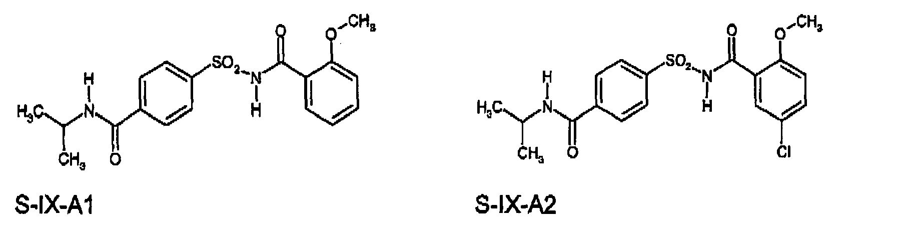 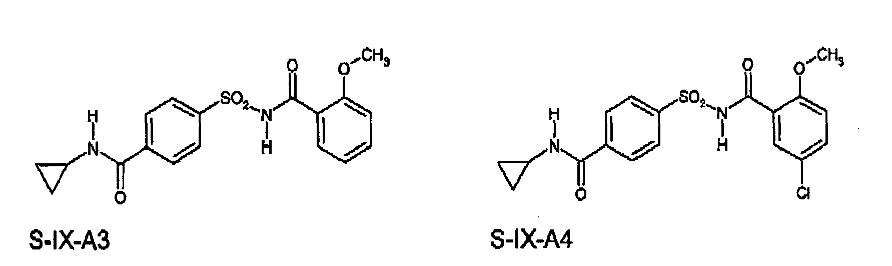 von denen wiederum die Verbindung S-IX-A3 als Safener ganz besonders bevorzugt ist.

Besonders bevorzugte Kombinationen von Herbiziden Wirkstoffen (A) wie in Tabelle (I) benannt und Safenern (B) sind solche, bei denen der Safener (B) ausgewählt ist aus der Gruppe von Safenem bestehend aus den Verbindungen der Formeln S-II-1 (Mefenpyr-diethyl), S-II-9 (Isoxadifen-ethyl), S-III-1 (Chloquintocet-mexyl), S-b-11 (Fenclorim), S-b-14 (Dymron), S-IX-A3 (4-Cyclopropylaminocarbonyl-N-(2-methoxybenzoyl)benzolsulfonamid, N-({4-[(cyclopropylamino)carbonyl]phenyl}sulfonyl)-2-methoxybenzamide, ganz besonders bevorzugt als Safener (B) sind Verbindungen S-II-1 und S-IX-A3).

Für die Anwendung in Reis besonders bevorzugt ist Isoxadifen-ethyl. Für die Anwendung in Getreide besonders bevorzugt sind Mefenpyr-diethyl, Cloquintocet-mexyl und 4-Cyclopropylaminocarbonyl-N-(2-methoxybenzoyl) benzolsulfonamid N-({4-[(cyclopropylamino)carbonyl] phenyl}sulfonyl)-2-methoxybenzamide, in Mais insbesondere Isoxadifen-ethyl und 4-Cyclopropylaminocarbonyl-N-(2-methoxy benzoyl)benzolsulfonamid N-({4-[(cyclopropylamino)carbonyl]phenyl} sulfonyl)-2-methoxy-benzamide. Für die Anwendung in Zuckerrohr bevorzugt ist Isoxadifen-ethyl.

Auch eine Mischung mit anderen bekannten Wirkstoffen, wie Fungiziden, Insektiziden, Akariziden, Nematiziden, Schutzstoffen gegen Vogelfraß, Pflanzennährstoffen und Bodenstrukturverbesserungsmitteln ist möglich.

Einige der Safener sind bereits als Herbizide bekannt und entfalten somit neben der Herbizidwirkung bei Schadpflanzen zugleich auch Schutzwirkung bei den Kulturpflanzen.
Die Gewichtsverhältnisse von Herbizid(mischung) zu Safener hängt im Allgemeinen von der Aufwandmenge an Herbizid und der Wirksamkeit des jeweiligen Safeners ab und kann innerhalb weiter Grenzen variieren, beispielsweise im Bereich von 200:1 bis 1:200, vorzugsweise 100:1 bis 1:100, insbesondere 20:1 bis 1:20. Die Safener können analog den Verbindungen der Formel (I) oder deren Mischungen mit weiteren Herbiziden/Pestiziden formuliert werden und als Fertigformulierung oder Tankmischung mit den Herbiziden bereitgestellt und angewendet werden.

Mit den äußeren Bedingungen wie Temperatur, Feuchtigkeit, der Art des verwendeten Herbizids, u.a. variiert die erforderliche Aufwandmenge der Verbindungen der Formel (I). Sie kann innerhalb weiter Grenzen schwanken, z.B. zwischen 0,001 und 10,0 kg/ha oder mehr Aktivsubstanz, vorzugsweise liegt sie jedoch zwischen 0,005 und 5 kg/ha.

Die erfindungsgemäßen Wirkstoffe können z.B. bei den folgenden Pflanzen verwendet werden:
Dikotyle Unkräuter der Gattungen: Abutilon, Amaranthus, Ambrosia, Anoda, Anthemis, Aphanes, Atriplex, Bellis, Bidens, Capsella, Carduus, Cassia, Centaurea, Chenopodium, Cirsium, Convolvulus, Datura, Desmodium, Emex, Erysimum, Euphorbia, Galeopsis, Galinsoga, Galium, Hibiscus, Ipomoea, Kochia, Lamium, Lepidium, Lindemia, Matricaria, Mentha, Mercurialis, Mullugo, Myosotis, Papaver, Pharbitis, Plantago, Polygonum, Portulaca, Ranunculus, Raphanus, Rorippa, Rotala, Rumex, Salsola, Senecio, Sesbania, Sida, Sinapis, Solanum, Sonchus, Sphenoclea, Stellaria, Taraxacum, Thlaspi, Trifolium, Urtica, Veronica, Viola, Xanthium.

Dikotyle Kulturen der Gattungen: Arachis, Beta, Brassica, Cucumis, Cucurbita, Helianthus, Daucus, Glycine, Gossypium, Ipomoea, Lactuca, Linum, Lycopersicon, Nicotiana, Phaseolus, Pisum, Solanum, Vicia.

Monokotyle Unkräuter der Gattungen: Aegilops, Agropyron, Agrostis, Alopecurus, Apera, Avena, Brachiaria, Bromus, Cenchrus, Commelina, Cynodon, Cyperus, Dactyloctenium, Digitaria, Echinochloa, Eleocharis, Eleusine, Eragrostis, Eriochloa, Festuca, Fimbristylis, Heteranthera, Imperata, Ischaemum, Leptochloa, Lolium, Monochoria, Panicum, Paspalum, Phalaris, Phleum, Poa, Rottboellia, Sagittaria, Scirpus, Setaria, Sorghum.

Monokotyle Kulturen der Gattungen: Allium, Ananas, Asparagus, Avena, Hordeum, Oryza, Panicum, Saccharum, Secale, Sorghum, Triticate, Triticum, Zea.

Die Verwendung der erfindungsgemäßen Wirkstoffe ist jedoch keineswegs auf diese Gattungen beschränkt, sondern erstreckt sich in gleicher Weise auch auf andere Pflanzen.

Die erfindungsgemäßen Wirkstoffe eignen sich in Abhängigkeit von der Konzentration zur Totalunkrautbekämpfung, z.B. auf Industrie- und Gleisanlagen und auf Wegen und Plätzen mit und ohne Baumbewuchs. Ebenso können die erfindungsgemäßen Wirkstoffe zur Unkrautbekämpfung in Dauerkulturen, z.B. Forst, Ziergehölz-, Obst-, Wein-, Citrus-, Nuss-, Bananen-, Kaffee-, Tee-, Gummi-, Ölpalm-, Kakao-, Beerenfrucht- und Hopfenanlagen, auf Zier- und Sportrasen und Weideflächen sowie zur selektiven Unkrautbekämpfung in einjährigen Kulturen eingesetzt werden.

Die erfindungsgemäßen Verbindungen der Formel (I) zeigen starke herbizide Wirksamkeit und ein breites Wirkungsspektrum bei Anwendung auf dem Boden und auf oberirdische Pflanzenteile. Sie eignen sich in gewissem Umfang auch zur selektiven Bekämpfung von monokotylen und dikotylen Unkräutern in monokotylen und dikotylen Kulturen, sowohl im Vorauflauf- als auch im Nachauflauf-Verfahren.

Die Herstellung und die Verwendung der erfindungsgemäßen Wirkstoffe geht aus den nachfolgenden Beispielen hervor.

Je nach Pflanzenarten bzw. Pflanzensorten, deren Standort und Wachstumsbedingungen (Böden, Klima, Vegetationsperiode, Ernährung) können durch die erfindungsgemäße Behandlung auch überadditive ("synergistische") Effekte auftreten. So sind beispielsweise erniedrigte Aufwandmenge und/oder Erweiterungen des Wirkungsspektrums und/oder eine Verstärkung der Wirkung der erfindungsgemäß verwendbaren Stoffe und Mittel - auch in Kombination mit anderen agrochemischen Wirkstoffen, besseres Wachstum der Kulturpflanzen, erhöhte Toleranz der Kulturpflanzen gegenüber hohen oder niedrigen Temperaturen, erhöhte Toleranz der Kulturpflanzen gegen Trockenheit oder gegen Wasser- bzw. Bodensalzgehalt, erhöhte Blühleistung, erleichterte Ernte, Beschleunigung der Reife, höhere Ernte-erträge, höhere Qualität und/oder höherer Ernährungswert der Ernteprodukte, höhere Lagerfähigkeit und/oder Bearbeitbarkeit der Ernteprodukte möglich, die über die eigentlich zu erwartenden Effekte hinausgehen.

Zu den bevorzugten erfindungsgemäß zu behandelnden transgenen (gentechnologisch erhaltenen) Pflanzen bzw. Pflanzensorten gehören alle Pflanzen, die durch die gentechnologische Modifikation genetisches Material erhielten, welches diesen Pflanzen besondere vorteilhafte wertvolle Eigenschaften ("Traits") verleiht. Beispiele für solche Eigenschaften sind besseres Pflanzenwachstum, erhöhte Toleranz gegenüber hohen oder niedrigen Temperaturen, erhöhte Toleranz gegen Trockenheit oder gegen Wasser- bzw. Bodensalzgehalt, erhöhte Blühleistung, erleichterte Ernte, Beschleunigung der Reife, höhere Ernteerträge, höhere Qualität und/oder höherer Emährungswert der Ernteprodukte, höhere Lagerfähigkeit und/oder Bearbeitbarkeit der Ernteprodukte. Weitere und besonders hervorgehobene Beispiele für solche Eigenschaften sind eine erhöhte Abwehr der Pflanzen gegen tierische und mikrobielle Schädlinge, wie gegenüber Insekten, Milben, pflanzenpathogenen Pilzen, Bakterien und/oder Viren sowie eine erhöhte Toleranz der Pflanzen gegen bestimmte herbizide Wirkstoffe. Als Beispiele transgener Pflanzen werden die wichtigen Kulturpflanzen, wie Getreide (Weizen, Reis), Mais, Soja, Kartoffel, Baumwolle, Raps sowie Obstpflanzen (mit den Früchten Äpfel, Birnen, Zitrusfrüchten und Weintrauben) erwähnt, wobei Mais, Soja, Kartoffel, Baumwolle und Raps besonders hervorgehoben werden. Als Eigenschaften ("Traits") werden besonders hervorgehoben die erhöhte Abwehr der Pflanzen gegen Insekten durch in den Pflanzen entstehende Toxine, insbesondere solche, die durch das genetische Material aus Bacillus Thuringiensis (z.B. durch die Gene CryIA(a), CryIA(b), CryIA(c), CryIIA, CryIIIA, CryIIIB2, Cry9c Cry2Ab, Cry3Bb und CryIF sowie deren Kombinationen) in den Pflanzen erzeugt werden (im folgenden "Bt Pflanzen"). Als Eigenschaften ("Traits") werden auch besonders hervorgehoben die erhöhte Abwehr von Pflanzen gegen Pilze, Bakterien und Viren durch Systemische Akquirierte Resistenz (SAR), Systemin, Phytoalexine, Elicitoren sowie Resistenzgene und entsprechend exprimierte Proteine und Toxine. Als Eigenschaften ("Traits") werden weiterhin besonders hervorgehoben die erhöhte Toleranz der Pflanzen gegenüber bestimmten herbiziden Wirkstoffen, beispielsweise Imidazolinonen, Sulfonylharnstoffen, Glyphosate oder Phosphinothricin (z.B. "PAT"-Gen). Die jeweils die gewünschten Eigenschaften ("Traits") verleihenden Gene können auch in Kombinationen miteinander in den transgenen Pflanzen vorkommen. Als Beispiele für "Bt Pflanzen" seien Maissorten, Baumwollsorten, Sojasorten und Kartoffelsorten genannt, die unter den Handelsbezeichnungen YIELD GARD® (z.B. Mais, Baumwolle, Soja), KnockOut® (z.B. Mais), StarLink® (z.B. Mais), Bollgard® (Baumwolle), Nucotn® (Baumwolle) und NewLeaf® (Kartoffel) vertrieben werden. Als Beispiele für Herbizid-tolerante Pflanzen seien Maissorten, Baumwollsorten und Sojasorten genannt, die unter den Handelsbezeichnungen Roundup Ready® (Toleranz gegen Glyphosate z.B. Mais, Baumwolle, Soja), Liberty Link® (Toleranz gegen Phosphinothricin, z.B. Raps), IMI® (Toleranz gegen Imidazolinone) und STS® (Toleranz gegen Sulfonylharnstoffe z.B. Mais) vertrieben werden. Als Herbizid-resistente (konventionell auf Herbizid-Toleranz gezüchtete) Pflanzen seien auch die unter der Bezeichnung Clearfield® vertriebenen Sorten (z.B. Mais) erwähnt. Selbstverständlich gelten diese Aussagen auch für in der Zukunft entwickelte bzw. zukünftig auf den Markt kommende Pflanzensorten mit diesen oder zukünftig entwickelten genetischen Eigenschaften ("Traits").
Die aufgeführten Pflanzen können besonders vorteilhaft erfindungsgemäß mit den Verbindungen der allgemeinen Formel (I) bzw. den erfindungsgemäßen Wirkstoffmischungen behandelt werden, wobei zusätzlich zu der guten Bekämpfung der Unkrautpflanzen die oben genannten synergistischen Effekte mit den transgenen Pflanzen oder Pflanzensorten auftreten. Die bei den Wirkstoffen bzw. Mischungen oben angegebenen Vorzugsbereiche gelten auch für die Behandlung dieser Pflanzen. Besonders hervorgehoben sei die Pflanzenbehandlung mit den im vorliegenden Text speziell aufgeführten Verbindungen bzw. Mischungen.
Die Herstellung und die Verwendung der erfindungsgemäßen Wirkstoffe geht aus den nachfolgenden Beispielen hervor. Herstellungsbeispiele:

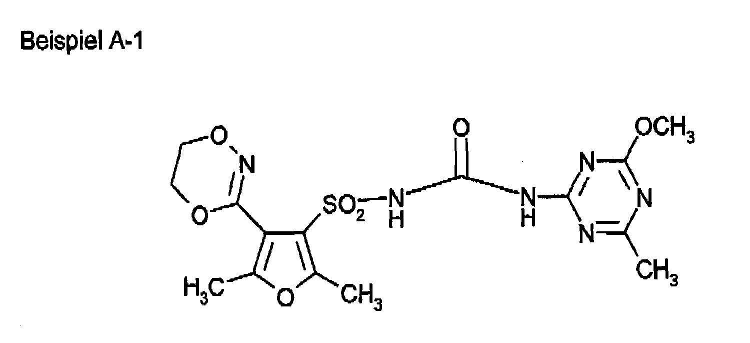

Zu einer Lösung von 0,545 g (2,096 mmol) 2-Phenoxycarbonylamino-4-methoxy-6-methyl-1,3,5-triazin in 40 ml Acetonitril werden bei Raumtemperatur (ca. 20 °C) nacheinander 0,60 g (2,305 mmol) 4-(5,6-dihydro-[1,4,2]-dioxazin-3-yl)-2,5-dimethyl-furyl-3-sulfonamid und 0,35 g DBU (2,305 mmol) gegeben. Nach 12 Stunden Rühren bei Raumtemperatur wird das Lösungsmittel im Wasserstrahlvakuum entfernt, mit 100 ml Methylenchlorid versetzt, mit 5%-iger Salzsäure und mit Wasser gewaschen, über Natriumsulfat getrocknet und schließlich das zugegebene Lösungsmittel im Wasserstrahlvakuum entfernt. Der Rückstand wird aus Isopropanol umkristallisiert, abfiltriert und getrocknet.

Man erhält 0,40 g (45% der Theorie) N-(4-Methoxy-6-methy-1,3,5-triazin-2-yl)-N'-(4-(5,6-dihydro-[1,4,2]-dioxazin-3-yl)-2,5-dimethyl-fur-3-yl-sulfonyl)-harnstoff vom Schmelzpunkt 206 °C.

Analog zu Beispiel A-1 sowie entsprechend der allgemeinen Beschreibung des erfindungsgemäßen Herstellungsverfahrens können beispielsweise auch die in der nachstehenden Tabelle 1 aufgeführten Verbindungen der allgemeinen Formel (I) hergestellt werden.

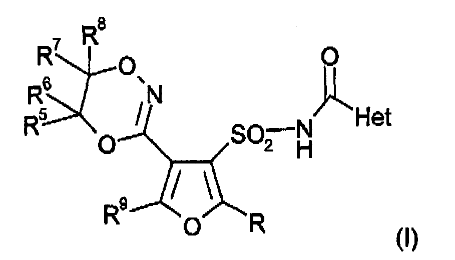

**Tabelle 1: Beispiele für die Verbindungen der Formel (I)**

| | | | | | | | |
|---|---|---|---|---|---|---|---|
| 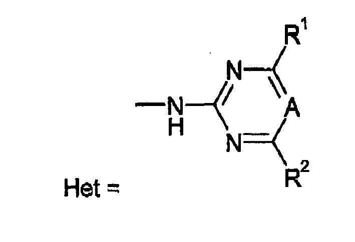 | | | | | | | |

| Bsp. -Nr. | A | R¹ | R² | R | R⁹ | R⁵, R⁶, R⁷, R⁸ | Schmelzpunkt (°C) |
|---|---|---|---|---|---|---|---|
| A-2 | CH | OCH₃ | OCH₃ | CH₃ | CH₃ | H | 208 |
| A-3 | CH | OCH₃ | Cl | CH₃ | CH₃ | H | 199 |
| A-4 | N | OCH₃ | OCH₃ | CH₃ | CH₃ | H | 215 |
| A-5 | CH | OCH₃ | CH₃ | CH₃ | CH₃ | H | |
| A-6 | CH | CH₃ | CH₃ | CH₃ | CH₃ | H | |
| A-7 | N | CH₃ | SCH₃ | CH₃ | CH₃ | H | |
| A-8 | N | OCH₃ | 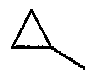 | CH₃ | CH₃ | H | |
| A-9 | N | CH₃ | OC₂H₅ | CH₃ | CH₃ | H | |
| A-10 | N | OCH₃ | C₂H₅ | CH₃ | CH₃ | H | |
| A-11 | N | OCH₃ | CH₃ | CH₃ | CH₃ | H | 206 |

### Beispiel B-1

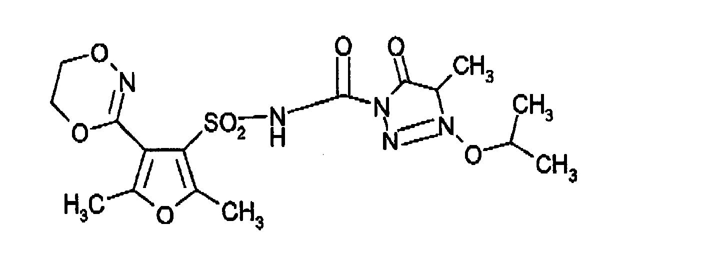

Zu einer Lösung von 0,581 g (2,096 mmol) 5-Isopropyloxy-4-methyl-2-phenoxy-carbonyl-2,4-dihydro-3H-1,2,4-triazol-3-on in 40 ml Acetonitril werden bei Raumtemperatur (ca. 20 °C) nacheinander 0,60 g (2,305 mmol) 4-(5,6-dihydro-[1,4,2]-dioxazin-3-yl)-2,5-dimethyl-furyl-3-sulfonamid und 0,35 g DBU (2,305 mmol) gegeben. Nach 12 Stunden Rühren bei Raumtemperatur wird das Lösungsmittel im Wasserstrahlvakuum entfernt, mit 100 ml Methylenchlorid versetzt, mit 5%-ige Salzsäure und mit Wasser gewaschen, über Natriumsulfat getrocknet und schließlich das zugegebene Lösungsmittel im Wasserstrahlvakuum entfernt. Der Rückstand wird aus Isopropanol umkristallisiert und abfiltriert und getrocknet.

Man erhält 0,45 g (48% der Theorie) 5-Isopropyloxy-4-methyl-2-[[4-(5,6-dihydro-[1,4,2]-dioxazin-3-yl)-2,5-dimethyl-fur-3-yl]-sulfonyl-amino-carbonyl]-2,4-dihydro-3H-1,2,4-triazol-3-on vom Schmelzpunkt 183 °C.

Analog zu Beispiel B-1 sowie entsprechend der allgemeinen Beschreibung des erfindungsgemäßen Herstellungsverfahrens können beispielsweise auch die in der nachstehenden Tabelle 2 aufgeführten Verbindungen der allgemeinen Formel (I) hergestellt werden.

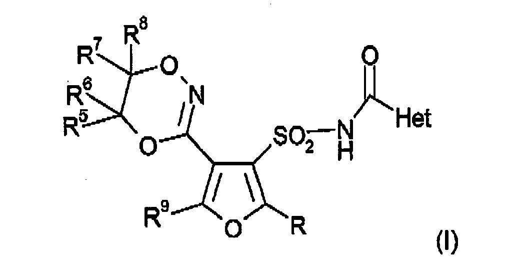

**Tabelle 2: Beispiele für die Verbindungen der Formel (I)**

| | | | | | | |
|---|---|---|---|---|---|---|
| 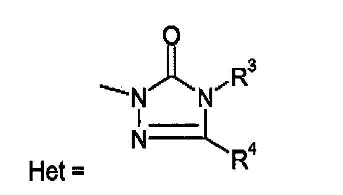 | | | | | | |

| Bsp. -Nr. | R³ | R⁴ | R | R⁹ | R⁵, R⁶, R⁷, R⁸ | Schmelzpunkt (°C) |
|---|---|---|---|---|---|---|
| B-2 | CH₃ | OCH₃ | CH₃ | CH₃ | H | 203 |
| B-3 | CH₃ | OCH₂CH₃ | CH₃ | CH₃ | H | 156 |
| B-4 | CH₃ | O-(n-Propyl) | CH₃ | CH₃ | H | 183 |
| B-5 | 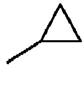 | OCH₃ | CH₃ | CH₃ | H | 192 |
| B-6 | 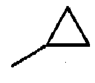 | OCH₂CH₃ | CH₃ | CH₃ | H | 178 |
| B-7 | 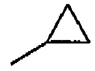 | O-(n-Propyl) | CH₃ | CH₃ | H | 174 |
| B-8 | 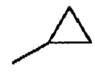 | O-(i-Propyl) | CH₃ | CH₃ | H | 194 |
| B-9 | CH₃ | OCH₂CF₃ | CH₃ | CH₃ | H | 179 |
| B-10 | 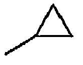 | OCH₂CF₃ | CH₃ | CH₃ | H | 188 |
| B-11 | CH₃ | O-(i Propyl) | CH₃ | CH₃ | H | 196 |

### Ausgangsstoffe der Formel (III):

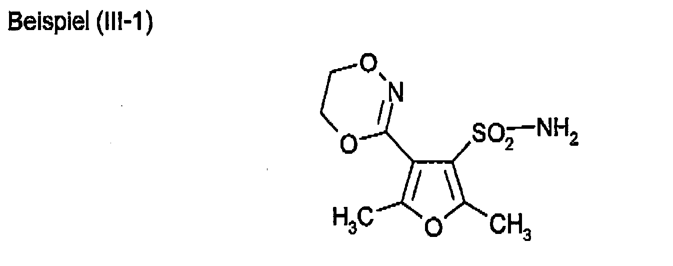

44,5 g (0,645 mol) Hydroxylamin-Hydrochlorid werden in 320 ml Methanol suspendiert und bei Raumtemperatur (ca. 20 °C) mit einer Lösung aus 72,2 g (1.29 mol) Kaliumhydroxid in 320 ml Methanol versetzt. Bei Raumtemperatur werden 50 g (0,2157mol) 2,5-Dimethyl-3-sulfamoyl-furyl-4-carbonsäure-methylester portionsweise hinzugegeben und für 16 Stunden bei 40 °C gerührt. Nach Abkühlen auf Raumtemperatur werden 29,7 g (0,215 mol) Kaliumcarbonat zugegeben und 181,6 g (0,97 mol) 1,2-Dibromethan zugetropft. Dann wird für weitere 12 Stunden bei 60° C gerührt. Nach Abkühlen auf Raumtemperatur wird der ausgefallene Feststoff abgesaugt und das Lösungsmittel im Wasserstrahlvakuum abgezogen und der verbliebene Rückstand mit 400 ml Methylenchlorid gelöst, dreimal mit Wasser gewaschen, die organische Phase abgetrennt, über Magnesiumsulfat getrocknet, filtriert und eingeengt. Der verbleibende Rückstand wird an Kieselgel mit Essigsäureethylester/n-Hexan im Verhältnis 1 : 2 chromatographisch gereinigt.

Man erhält 26,8 g (48% der Theorie) 4-(5,6-dihydro-[1,4,2]-dioxazin-3-yl)-2,5-dimethyl-furyl-3-sulfonamid vom Schmelzpunkt 162°C

Analog zu Beispiel (III-1) sowie entsprechend der allgemeinen Beschreibung des erfindungsgemäßen Herstellungsverfahrens können beispielsweise auch die in der nachstehenden Tabelle 3 aufgeführten Verbindungen der allgemeinen Formel (III) hergestellt werden.

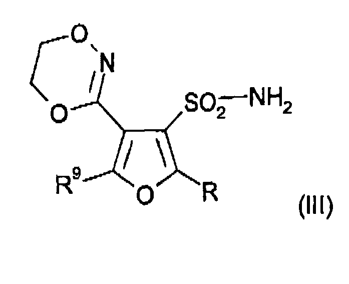

**Tabelle 3: Beispiele für die Verbindungen der Formel (III)**

| Bsp.-Nr. | R | R⁹ |
|---|---|---|
| III-2 | CH₃ | H |
| III-3 | C₂H₅ | H |
| III-4 | C₃H₇-n | H |
| III-5 | C₃H₇-i | H |
| III-6 | F | H |
| III-7 | Cl | H |
| III-8 | Br | H |
| III-9 | I | H |
| III-10 | OCH₃ | H |
| III-11 | C₂H₅ | CH₃ |
| III-12 | C₃H₇-n | CH₃ |
| III-13 | C₃H₇-i | CH₃ |
| III-14 | F | CH₃ |
| III-15 | Cl | CH₃ |
| III-16 | Br | CH₃ |
| III-17 | I | CH₃ |
| III-18 | OCH₃ | CH₃ |

### C. Biologische Beispiele

### 1. Unkrautwirkung im Vorauflauf

Samen bzw. Rhizomstücke von mono- und dikotylen Unkrautpflanzen wurden in Papptöpfen in sandiger Lehmerde ausgelegt und mit Erde abgedeckt. Die in Form von benetzbaren Pulvern oder Emulsionskonzentraten formulierten erfindungsgemäßen Verbindungen wurden dann als wässrige Suspensionen bzw. Emulsionen mit einer Wasseraufwandmenge von umgerechnet 600 bis 800 l/ha in unterschiedlichen Dosierungen auf die Oberfläche der Abdeckerde appliziert.

Nach der Behandlung wurden die Töpfe im Gewächshaus aufgestellt und unter guten Wachstumsbedingungen für die Unkräuter gehalten. Die optische Bonitur der Pflanzen- bzw. Auflaufschäden erfolgte nach dem Auflaufen der Versuchspflanzen nach einer Versuchszeit von 3 bis 4 Wochen im Vergleich zu unbehandelten Kontrollen. Wie die Ergebnisse zeigen, weisen erfindungsgemäße Verbindungen eine gute herbizide Vorauflaufwirksamkeit gegen ein breites Spektrum von Ungräsern und Unkräutern auf.
Beispielsweise haben die Verbindungen A-1, A-3, A-6, A-7 und A-9 sehr gute Wirkung gegen Unkräuter bei einer Aufwandmenge von 0.08 kg und weniger Aktivsubstanz pro Hektar.

### 2. Unkrautwirkung im Nachauflauf

Samen bzw. Rhizomstücke von mono- und dikotylen Unkräutern wurden in Plastiktöpfen in sandigem Lehmboden ausgelegt, mit Erde abgedeckt und im Gewächshaus unter guten Wachstumsbedingungen angezogen. Drei Wochen nach der Aussaat wurden die Versuchspflanzen im Dreiblattstadium behandelt. Die als Spritzpulver bzw. als Emulsionskonzentrate formulierten erfindungsgemäßen Verbindungen wurden in verschiedenen Dosierungen mit einer Wasseraufwandmenge von umgerechnet 600 bis 800 l/ha auf die grünen Pflanzenteile gesprüht. Nach ca. 3 bis 4 Wochen Standzeit der Versuchspflanzen im Gewächshaus unter optimalen Wachstumsbedingungen wurde die Wirkung der Präparate optisch im Vergleich zu unbehandelten Kontrollen bonitiert. Die erfindungsgemäßen Mittel weisen auch im Nachauflauf eine gute herbizide Wirksamkeit gegen ein breites Spektrum wirtschaftlich wichtiger Ungräser und Unkräuter auf. Beispielsweise haben die Verbindungen gemäß Herstellungsbeispiel A-1, A-3, A-6, A-7 und A-9 sehr gute Wirkung gegen Unkräuter bei einer Aufwandmenge von 0.08 kg und weniger Aktivsubstanz pro Hektar.

### 3. Kulturpflanzenverträglichkeit

In weiteren Versuchen im Gewächshaus wurden Samen einer größeren Anzahl von Kulturpflanzen und Unkräutern in sandigem Lehmboden ausgelegt und mit Erde abgedeckt. Ein Teil der Töpfe wurde sofort wie unter Abschnitt 1 beschrieben behandelt, die übrigen im Gewächshaus aufgestellt, bis die Pflanzen zwei bis drei echte Blätter entwickelt haben und dann wie unter Abschnitt 2 beschrieben mit den erfindungsgemäßen Verbindungen in unterschiedlichen Dosierungen besprüht. Vier bis fünf Wochen nach der Applikation und Standzeit im Gewächshaus wurde mittels optischer Bonitur festgestellt, dass erfindungsgemäße Verbindungen Gramineen-Kulturen wie Gerste, Weizen oder Reis im Vor- und Nachauflaufverfahren selbst bei hohen Wirkstoffdosierungen ungeschädigt ließen. Die erfindungsgemäßen Verbindungen zeigen teilweise eine hohe Selektivität und eignen sich deshalb zur Bekämpfung von unerwünschten Pflanzenwuchs in landwirtschaftlichen Kulturen.

## Patentansprüche

1. Verbindungen der allgemeinen Formel (I) 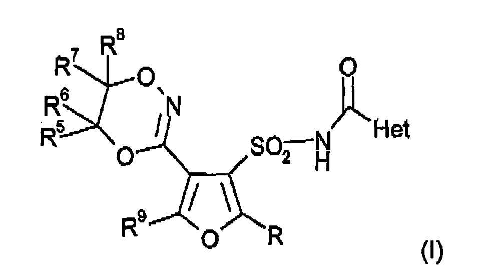 in welcher
Het für 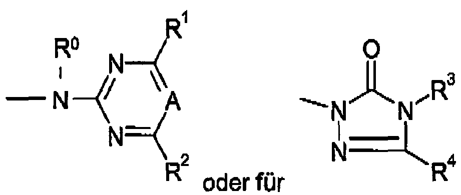 steht,
A für Stickstoff oder eine CH-Gruppierung steht,
R für Wasserstoff, Cyano, Nitro, Halogen, für jeweils gegebenenfalls durch Cyano, Halogen oder (C₁-C₄)-Alkoxy substituiertes Alkyl, Alkoxy, Alkoxycarbonyl, Alkylthio, Alkylsulfinyl oder Alkylsulfonyl mit jeweils 1 bis 6 Kohlenstoffatomen in der Alkylgruppe, oder für jeweils gege-benenfalls durch Cyano oder Halogen substituiertes Alkenyl, Alkinyl, Alkenyloxy oder Alkinyloxy mit jeweils 2 bis 6 Kohlenstoffatomen in der Alkenyl- oder Alkinylgruppe steht,
R⁰ für Wasserstoff, oder für (C₁-C₄)-Alkyl steht,
R¹ für Wasserstoff, für Halogen, für jeweils gegebenenfalls durch Cyano, Halogen oder (C₁-C₄)-Alkoxy substituiertes Alkyl, Alkoxy, Alkylthio, Alkylamino oder Dialkylamino mit jeweils 1 bis 4 Kohlenstoffatomen in den Alkylgruppen, oder für jeweils gegebenenfalls durch Cyano, Halogen, (C₁-C₄)-Alkyl oder (C₁-C₄)-Alkoxy substituiertes Phenoxy, Oxetanyloxy, Furyloxy oder Tetrahydrofuryloxy steht,
R² für Wasserstoff, für Halogen, für jeweils gegebenenfalls durch Cyano, Halogen oder (C₁-C₄)-Alkoxy substituiertes Alkyl, Alkoxy, Alkylthio, Alkylamino oder Dialkylamino mit jeweils 1 bis 4 Kohlenstoffatomen in den Alkylgruppen, oder für jeweils gegebenenfalls durch Cyano, Halogen, (C₁-C₄)-Alkyl oder (C₁-C₄)-Alkoxy substituiertes Phenoxy, Oxetanyloxy, Furyloxy oder Tetrahydrofuryloxy steht,
R³ für Wasserstoff, Hydroxy, Amino, Cyano, für (C₂-C₁₀)-kylidenamino, für gegebenenfalls durch Fluor, Chlor, Brom, Cyano, (C₁-C₄)-Alkoxy, (C₁-C₄)-Alkyl-carbonyl oder (C₁-C₄)-Alkoxy-carbonyl substituiertes Alkyl mit 1 bis 6 Kohlenstoffatomen, für jeweils gegebenenfalls durch Fluor, Chlor und/oder Brom substituiertes Alkenyl oder Alkinyl mit jeweils 2 bis 6 Kohlenstoffatomen, für jeweils gegebenenfalls durch Fluor, Chlor, Brom, Cyano, (C₁-C₄)-Alkoxy oder (C₁-C₄)-Alkoxy-carbonyl substituiertes Alkoxy, Alkylamino oder Alkyl-carbonylamino mit jeweils 1 bis 6 Kohlenstoffatomen in der Alkylgruppe, für Alkenyloxy mit 3 bis 6 Kohlenstoffatomen, für Dialkylamino mit jeweils 1 bis 4 Kohlenstoffatomen in den Alkylgruppen, für jeweils gegebenenfalls durch Fluor, Chlor, Brom, Cyano und/oder (C₁-C₄)-Alkyl substituiertes Cycloalkyl, Cycloalkylamino oder Cycloalkylalkyl mit jeweils 3 bis 6 Kohlenstoffatomen in der Alkylgruppe und gegebenenfalls 1 bis 4 Kohlenstoffatomen im Alkylteil, oder für jeweils gegebenenfalls durch Fluor, Chlor, Brom, Cyano, Nitro, (C₁-C₄)-Alkyl, Trifluormethyl und/oder (C₁-C₄)-Alkoxy substituiertes Aryl oder Arylalkyl mit jeweils 6 oder 10 Kohlenstoffatomen in der Arylgruppe und gegebenenfalls 1 bis 4 Kohlenstoffatomen im Alkylteil steht,
R⁴ für Wasserstoff, Hydroxy, Mercapto, Amino, Cyano, Fluor, Chlor, Brom, lod, für gegebenenfalls durch Fluor, Chlor, Brom, Cyano, (C₁-C₄)-Alkoxy, (C₁-C₄)-Alkyl-carbonyl oder (C₁-C₄)-Alkoxy-carbonyl substituiertes Alkyl mit 1 bis 6 Kohlenstoffatomen, für jeweils gegebenenfalls durch Fluor, Chlor und/oder Brom substituiertes Alkenyl oder Alkinyl mit jeweils 2 bis 6 Kohlenstoffatomen, für jeweils gegebenenfalls durch Fluor, Chlor, Cyano, (C₁-C₄)-Alkoxy oder (C₁-C₄)-Alkoxy-carbonyl sub-stituiertes Alkoxy, Alkylthio, Alkylamino oder Alkyl-carborlylamino mit jeweils 1 bis 6 Kohlenstoffatomen in der Alkylgruppe, für Alkenyloxy, Alkinyloxy, Alkenylthio, Alkinylthio, Alkenylamino oder Alkinylamino mit jeweils 3 bis 6 Kohlenstoffatomen in der Alkenyl- oder Alkinylgruppe, für Dialkylamin mit jeweils 1 bis 4 Kohlenstoffatomen in den Alkylgruppen, für jeweils gegebenenfalls durch Fluor, Chlor, Brom, Cyano und/oder (C₁-C₄)-Alkyl substituiertes Cycloalkyl, Cycloalkenyl, Cyclo-alkyloxy, Cycloalkylthio, Cycloalkylamino, Cycloalkylalkyl, Cycloalkylalkoxy, Cycloalkylalkylthio oder Cycloalkylalkylamino mit jeweils 3 bis 6 Kohlenstoffatomen in der Cycloalkyl- bzw. Cycloalkenylgruppe und gegebenenfalls 1 bis 4 Kohlenstoffatomen im Alkylteil, oder für jeweils gegebenenfalls durch Fluor, Chlor, Brom, Cyano, Nitro, (C₁-C₄)-Alkyl, Tri-fluormethyl, (C₁-C₄)-Alkoxy und/oder (C₁-C₄)-Alkoxy-carbonyl substituiertes Aryl, Arylalkyl, Aryloxy, Arylalkoxy, Arylthio, Arylalkylthio, Arylamino oder Arylalkylamino mit jeweils 6 oder 10 Kohlenstoffatomen in der Arylgruppe und gegebenenfalls 1 bis 4 Kohlenstoffatomen im Alkylteil steht, und
R⁵, R⁶, R⁷ und R⁸ unabhängig voneinander für Wasserstoff, Halogen, Cyano oder Thiocyanato oder für gegebenenfalls durch Halogen substituiertes Alkyl, Alkoxy, Alkylthio, Alkylsulfinyl, Alkylsulfonyl Alkylamino, Alkylcarbonyl, Alkoxycarbonyl oder Alkylaminocarbonyl mit jeweils 1 bis 3 Kohlenstoffatomen im Alkylteil stehen,
R⁹ für Wasserstoff, Cyano, Nitro, Halogen, für jeweils gegebenenfalls durch Cyano, Halogen oder (C₁-C₄)Alkoxy substituiertes Alkyl, Alkoxy, Alkoxycarbonyl, Alkylthio, Alkylsulfinyl oder Alkylsulfonyl mit jeweils 1 bis 6 Kohlenstoffatomen in der Alkylgruppe, oder für jeweils gegebenenfalls durch Cyano oder Halogen substituiertes Alkenyl, Alkinyl, Alkenyloxy oder Alkinyloxy mit jeweils 2 bis 6 Kohlenstoffatomen in der Alkenyl- oder Alkinylgruppe steht,
sowie Salze von Verbindungen der Formel (I).

2. Verbindungen gemäß Anspruch 1, **dadurch gekennzeichnet, dass**
R für Wasserstoff, Cyano, Fluor, Chlor, Brom, für jeweils gegebenenfalls durch Cyano, Fluor, Chlor, Methoxy oder Ethoxy substituiertes Methyl, Ethyl, n- oder i-Propyl, n-, i-, s- oder t-Butyl, Methoxy, Ethoxy, n- oder i- Propoxy, Methoxycarbonyl, Ethoxycarbonyl, n- oder i-Propoxycarbonyl, , Methylthio, Ethylthio, n- oder i-Propylthio, Methylsulfinyl, Ethylsulfinyl, Methylsulfonyl oder Ethylsulfonyl, oder für jeweils gegebenenfalls durch Cyano, Fluor oder Chlor substituiertes Propenyl, Butenyl, Propinyl, Butinyl, Propenyloxy, Butenyloxy, Propinyloxy oder Butinyloxy steht,
R⁰ für Wasserstoff steht,
R¹ für Fluor, Chlor, Brom, lod, oder für jeweils gegebenenfalls durch Cyano, Fluor, Chlor, Methoxy oder Ethoxy substituiertes Methyl, Ethyl, n- oder i-Propyl, Methoxy, Ethoxy, n- oder i-Propoxy, Methylthio, Ethylthio, n- oder i-Propylthio, Methylamino, Ethylamino, n- oder i-Propylamino, Dimethylamino oder Diethylamino steht,
R² für Fluor, Chlor, Brom, lod, oder für jeweils gegebenenfalls durch Cyano, Fluor, Chlor, Methoxy oder Ethoxy .substituiertes Methyl, Ethyl, n- oder 1-Propyl, Methoxy, Ethoxy, n- oder i-Propoxy, Methylthio, Ethylthio, n- oder i-Propylthio, Methylamino, Ethylamino, n- oder i-Propylamino, Dimethylamino oder Diethylamino steht,
R³ für Wasserstoff, Hydroxy, Amino, für jeweils gegebenenfalls durch Fluor, Chlor, Cyano, Methoxy oder Ethoxy substituiertes Methyl, Ethyl, n- oder i-Propyl, n-, i-, s- oder t-Butyl, für jeweils gegebenenfalls durch Fluor, Chlor und/oder Brom substituiertes Ethenyl, Propenyl, Butenyl, Propinyl oder Butinyl, für jeweils gegebenenfalls durch Fluor, Chlor, Cyano, Methoxy oder Ethoxy substituiertes Methoxy, Ethoxy, n- oder i-Propoxy, n-, i-, s- oder t-Butoxy, Methylamino, Ethylamino, n- oder i-Propylamino, n-, i-, s- oder t-Butylamino, für Propenyloxy oder Butenyloxy, für Dimethylamino oder Diethylamino, für jeweils gegebenenfalls durch Fluor, Chlor, Methyl und/oder Ethyl substituiertes Cyclopropyl, Cyclobutyl, Cyclopentyl, Cyclohexyl, Cyclopropylamino, Cyclobutylamino, Cyclopentylamino, Cyclohexylamino, Cyclopropylmethyl, Cyclobutylmethyl, Cyclopentylmethyl oder Cyclohexylmethyl, oder für jeweils gegebenenfalls durch Fluor, Chlor, Methyl, Trifluormethyl und/oder Methoxy substituiertes Phenyl oder Benzyl steht,
R⁴ für Wasserstoff, Hydroxy, Mercapto, Amino, Cyano, Fluor, Chlor, Brom, für jeweils gegebenenfalls durch Fluor, Chlor, Cyano, Methoxy, Ethoxy, n- oder i-Propoxy, Acetyl, Propionyl, n- oder i-Buyroyl, Methoxy-carbonyl, Ethoxycarbonyl, n- oder i-Propoxy-carbonyl substituiertes Methyl, Ethyl, n- oder i-Propyl, n-, i-, s- oder t-Butyl, für jeweils gegebenenfalls durch Fluor, Chlor und/oder Brom substituiertes Ethenyl, Propenyl, Butenyl, Ethinyl, Propinyl oder Butinyl, für jeweils gegebenenfalls durch Fluor, Chlor, Cyano, Methoxy, Ethoxy, n- oder i-Propoxy, Methoxycarbonyl, Ethoxycarbonyl, n- oder i-Propoxy-carbonyl substituiertes Methoxy, Ethoxy, n- oder i-Propoxy, n-, i-, s- oder t-Butoxy, Methylthio, Ethylthio, n- oder i-Propylthio, n-, i-, s- oder t-Butylthio, Methylamino, Ethylamino, n- oder i-Propylamino, n-, i-, s- oder t-Butylamino, Acetylamino oder Propionylamino, für Propenyloxy, Butenyloxy, Ethinyloxy, Propinyloxy, Butinyloxy, Propenylthio, Butenylthio, Propinylthio, Butinylthio, Propenylamino, Butenylamino, Propinylamino oder Butinylamino, für Dimethylamino, Diethylamino oder Dipropylamino, für jeweils gegebenenfalls durch Fluor, Chlor, Methyl und/oder Ethyl substituiertes Cyclopropyl, Cyclobutyl, Cyclopentyl, Cyclohexyl, Cyclopentenyl, Cyclohexenyl, Cyclopropyloxy, Cyclobutyloxy, Cyclopentyloxy, Cyclohexyloxy, Cyclopropylthio, Cyclobutylthio, Cyclopentylthio, Cyclohexylthio, Cyclopropylamino, Cyclobutylamino, Cyclopentylamino, Cyclohexylamino, Cyclopropylmethyl, Cyclobutylmethyl, Cyclopentylmethyl, Cyclohexylmethyl, Cyclopropylmethoxy, Cyclobutylmethoxy, Cyclopentylmethoxy, Cyclohexylmethoxy, Cyclo-propylmethylthio, Cyclobutylmethylthio, Cyclopentylmethylthio, Cyclo-hexylmethylthio, Cyclopropylmethylamino, Cyclobutylmethylamino, Cyclopentylmethylamino oder Cyclohexylmethylamino, oder für jeweils gegebenenfalls durch Fluor, Chlor, Brom, Methyl, Trifluormethyl, Methoxy oder Methoxy-carbonyl substituiertes Phenyl, Benzyl, Phenoxy, Benzyloxy, Phenylthio, Benzylthio, Phenylamino oder Benzyl-amino steht,
R⁵, R⁶, R⁷ und R⁸ stehen bevorzugt unabhängig voneinander für Wasserstoff oder Methyl stehen und
R⁹ für Wasserstoff, Cyano, Fluor, Chlor, Brom, für jeweils gegebenenfalls durch Cyano, Fluor, Chlor, Methoxy oder Ethoxy substituiertes Methyl, Ethyl, n- oder i-Propyl, n-, i-, s- oder t-Butyl, Methoxy, Ethoxy, n- oder i-Propoxy, Methoxycarbonyl, Ethoxycarbonyl, n- oder i-Propoxycarbonyl, Methylthio, Ethylthio, n- oder i-Propylthio, Methylsulfinyl, Ethylsulfinyl, Methylsulfonyl oder Ethylsulfonyl, oder für jeweils gegebenenfalls durch Cyano, Fluor oder Chlor substituiertes Propenyl, Butenyl, Propinyl, Butinyl, Propenyloxy, Butenyloxy, Propinyloxy oder Butinyloxy steht,
sowie die Natrium-, Kalium-, Magnesium-, Calcium-, Ammonium-, (C₁-C₄)-Alkyl-ammonium-, Di-((C₁-C₄)alkyl)-ammonium-, Tri-(C₁-C₄)-alkyl)-ammonium-, Tetra-((C₁-C₄)-alkyl)-ammonium, Tri-((C₁-C₄)-alkyl)-sulfonium-, C₅- oder C₆-Cycloalkyl-ammonium- und Di-((C₁-C₂)-alkyl)-benzyl-ammoniumSalze dieser Verbindungen.

3. Verbindungen gemäß Anspruch 1, **dadurch gekennzeichnet, dass**
R für Fluor, Chlor, Brom, für jeweils gegebenenfalls durch Cyano, Fluor, Chlor, Methoxy oder Ethoxy substituiertes Methyl, Ethyl, n- oder i-Propyl, n-, i-, s- oder t-Butyl, Methoxy, Ethoxy, n- oder i-Propoxy steht,
R⁰ für Wasserstoff steht,
R¹ für Fluor, Chlor, Brom, oder Iod, oder für gegebenenfalls durch Fluor oder Chlor substituiertes Methyl, Ethyl, n- oder i-Propyl, Methoxy, Ethoxy, n- oder i-Propoxy oder für Dimethylamino steht,
R² für Fluor, Chlor, Brom, oder Iod, oder für gegebenenfalls durch Fluor oder Chlor substituiertes Methyl, Ethyl, n- oder i-Propyl, Methoxy, Ethoxy, n- oder i-Propoxy oder für Dimethylamino steht,
R³ für Methyl, Ethyl, n- oder i-Propyl oder Cyclopropyl steht,
R⁴ für Methoxy, Ethoxy, n- oder i-Propoxy, n-, i-, s- oder t-Butoxy steht, und
R⁵, R⁶, R⁷ und R⁸ für Wasserstoff oder Methyl stehen,
R⁹ für Wasserstoff, Methyl, Ethyl, n- oder i-Propyl steht sowie die Natrium-, Kalium-, Magnesium-, Calcium-, Ammonium-, (C₁-C₄)-Alkyl-ammonium-, Di-((C₁-C₄)-alkyl)-ammonium-, Tri-((C₁-C₄)-alkyl)-ammonium-, Tetra-((C₁-C₄)-alkyl)-ammonium-, Tri-((C₁-C₄)-alkyl)-sulfonium, C₅- oder C₆-Cycloalkyl-ammonium- oder Di-((C₁-C₂)-alkyl)-benzyl-ammoniumSalze von Verbindungen der Formel (I),

4. Verfahren zum Herstellen von Verbindungen gemäß Anspruch 1, **dadurch gekennzeichnet, dass**
(a) substituierte Aminoazine der allgemeinen Formel (II) 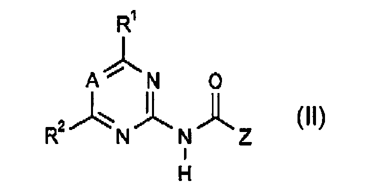 in welcher
A, R¹ und R² die in Anspruch 1 angegebene Bedeutung haben, und
Z für Halogen, Alkoxy oder Aryloxy steht,
mit Furanderivaten der allgemeinen Formel (III) 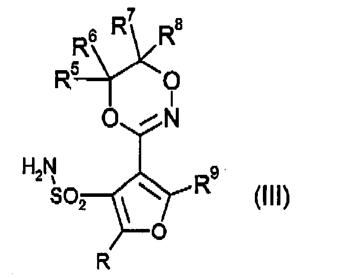 in welcher
R und R⁵ bis R⁹ die in Anspruch 1 angegebene Bedeutung haben, gegebenenfalls in Gegenwart eines Reaktionshilfsmittels und gegebenenfalls in Gegenwart eines Verdünnungsmittels umgesetzt werden,
oder (b) substituierte Furan-3-sulfonamide der allgemeinen Formel (III) 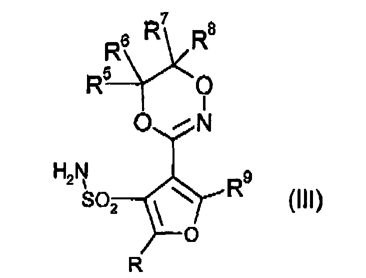 in welcher R und R⁵ bis R⁹ die in Anspruch 1 angegebene Bedeutung haben, mit substituierten Triazolinonen der allgemeinen Formel (IV) 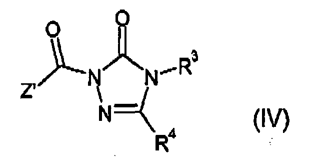 in welcher
R³ und R⁴ die in Anspruch 1 angegebene Bedeutung haben und
Z' für Halogen, Alkoxy, Aryloxy oder Arylalkoxy steht,
gegebenenfalls in Gegenwart eines Reaktionshilfsmittels und gegebenenfalls in Gegenwart eines Verdünnungsmittels umgesetzt werden,
oder (c) substituierte Aminoazine der allgemeinen Formel (II) 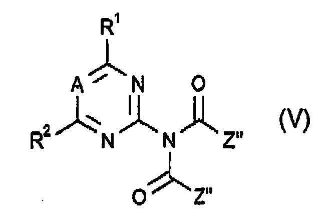 in welcher
A, R¹ und R² die oben angegebene Bedeutung haben, und
Z" für Halogen, Alkoxy oder Aryloxy steht,
mit Furanderivaten der allgemeinen Formel (III) 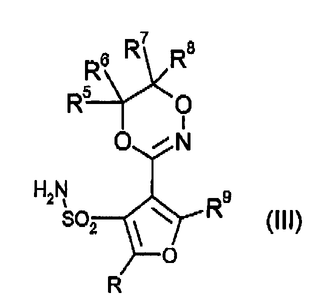 in welcher
R und R⁵ bis R⁹ die oben angegebene Bedeutung haben,
gegebenenfalls in Gegenwart eines Reaktionshilfsmittels und gegebenenfalls
in Gegenwart eines Verdünnungsmittels umsetzt,
oder (d) Aminoazine der allgemeinen Formel (VI) 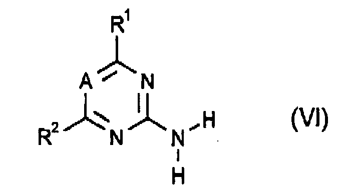 in welcher
A, R¹ und R² die oben angegebene Bedeutung haben, und
mit Furanderivaten der allgemeinen Formel (VII) 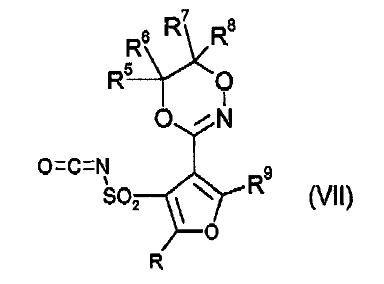 in welcher
R und R⁵ bis R⁸ die oben angegebene Bedeutung haben,
gegebenenfalls in Gegenwart eines Reaktionshilfsmittels und gegebenenfalls in Gegenwart eines Verdünnungsmittels umsetzt,
und gegebenenfalls die nach den Verfahren (a) oder (b) erhaltenen Verbindungen der Formel (I) nach üblichen Methoden in Salze überführt werden.

5. Verfahren zum Bekämpfen von unerwünschtem Pflanzenwuchs, **dadurch gekennzeichnet, dass** man mindestens eine Verbindung gemäß einem der Ansprüche 1 bis 3 auf unerwünschte Pflanzen und/oder ihren Lebensraum einwirken lässt.

6. Verwendung von mindestens einer Verbindung gemäß einem der Ansprüche 1 bis 3 zum Bekämpfen von unerwünschten Pflanzen.

7. Herbizides Mittel, **gekennzeichnet durch** einen Gehalt an einer Verbindung gemäß einem der Ansprüche 1 bis 3 und üblichen Streckmitteln und/oder oberflächenaktiven Mitteln.

8. Verbindungen der Formel (III) 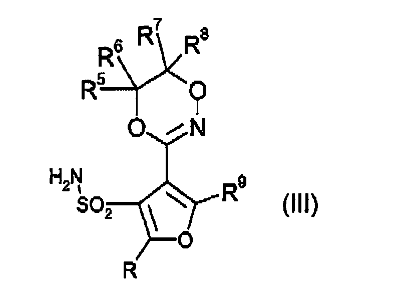 in welcher
R und R⁵ bis R⁹ die in Anspruch 1 angegebene Bedeutung haben.
